# EUROPEAN PATENT APPLICATION

(11) **EP 2 123 758 A1**
(43) Date of publication of application: **25.11.2009**
(21) Application number: 08156582.2
(22) Date of filing: 20.05.2008
(51) Int. Cl.: C12N 15/11

(54) **Influenza cap-leader sequence**

(71) Applicant: Wageningen Universiteit, 6701 BH Wageningen (NL)
(72) Inventor: The designation of the inventor has not yet been filed
(74) Representative: Hatzmann, Martin

(57) **Abstract**

The invention comprises a viral cap-leader sequence which comprises at least three bases complementary to the 3'-ultimate residues of a (-)ssRNA virus template sequence. Preferably said consensus sequence is represented by the general formula ^{7m}G (N)₆₋₁₀-(A/U/G)-A/U-AGC, more preferably ^{7m}G (N)₇₋₈-(A/U/G)-A/U-AGC. Further comprised are modified cap-leader sequences that are able to block elongation by the viral polymerase or cleavage by the viral endonuclase. Such modified cap-leader sequences comprise a 3'-terminal phosphate group, one or more phosphorothioate or phosphorodiamidate bonds or one ore more morpholino rings. Also pharmaceutical products comprising such a modified cap-leader sequence are disclosed.

Further, the invention comprises methods for inhibiting virus transcription, especially for influenza virus, using such a modified cap-leader sequence or pharmaceutical product.

SEQUENCE LISTING

<110> Wageningen Universiteit

<120> Influenza cap-leader sequence

<130> P83864EP00

<160> 115

<170> PatentIn version 3.3

<210> 1
<211> 12
<212> RNA
<213> Artificial

<220>
<223> consensus (n=6)

<220>
<221> modified_base
<222> (1)..(1)
<223> m7g

<220>
<221> misc_feature
<222> (2)..(7)
<223> n is a, c, g, or u

<400> 1
gnnnnnndwa gc 12

<210> 2
<211> 13
<212> RNA
<213> Artificial

<220>
<223> consensus (n=7)

<220>
<221> modified_base
<222> (1)..(1)
<223> m7g

<220>
<221> misc_feature
<222> (2)..(8)
<223> n is a, c, g, or u

<400> 2
gnnnnnnndw agc                                                    13

<210> 3
<211> 14
<212> RNA
<213> Artificial

<220>
<223> consensus (n=8)

<220>
<221> modified_base
<222> (1)..(1)
<223> m7g

<220>
<221> misc_feature
<222> (2)..(9)
<223> n is a, c, g, or u

<400> 3
gnnnnnnnnd wagc                                                   14

<210> 4
<211> 15
<212> RNA
<213> Artificial

<220>
<223> consensus (n=9)

<220>
<221> modified_base
<222> (1)..(1)
<223> m7g

<220>
<221> misc_feature
<222> (2)..(10)
<223> n is a, c, g, or u

<400> 4
gnnnnnnnnn dwagc                                                  15

<210> 5
<211> 16
<212> RNA
<213> Artificial

<220>
<223> consensus (n=10)

<220>
<221> modified_base
<222> (1)..(1)
<223> m7g

<220>
<221> misc_feature
<222> (2)..(11)
<223> n is a, c, g, or u

<400> 5
gnnnnnnnnn ndwagc 16

<210> 6
<211> 12
<212> RNA
<213> Influenza A virus

<400> 6
uucgcuucug cu 12

<210> 7
<211> 12
<212> RNA
<213> Influenza B virus

<400> 7
ccugcuuuug cu 12

<210> 8
<211> 27
<212> RNA
<213> Alfalfa mosaic virus

<400> 8
guauuaauac cauuuucaaa auauucc 27

<210> 9
<211> 27
<212> RNA
<213> Artificial

<220>
<223> mutant cap-leader

<400> 9
guauuaauaa caguuucaaa auauucc                                27

<210> 10
<211> 27
<212> RNA
<213> Artificial

<220>
<223> mutant cap-leader

<400> 10
guauuaauag cagcuucaaa auauucc                                27

<210> 11
<211> 25
<212> RNA
<213> Artificial

<220>
<223> mutant cap-leader

<400> 11
guauuaauau cagcaaaagc agggu                                  25

<210> 12
<211> 27
<212> RNA
<213> Artificial

<220>
<223> mutant cap-leader

<400> 12
guauuaauac cauuuucaaa auauucc                                27

<210> 13
<211> 27
<212> RNA
<213> Artificial

<220>
<223> mutant cap-leader

<400> 13
   guauuaauac gauuuucaaa auauucc                                 27

<210> 14
<211> 27

<212> RNA
<213> Artificial

<220>
<223> mutant cap-leader

<400> 14
guauuaauac aauuuucaaa auauucc 27

<210> 15
<211> 27
<212> RNA
<213> Artificial

<220>
<223> mutant cap-leader

<400> 15
guauuaauac uauuuucaaa auauucc 27

<210> 16
<211> 27
<212> RNA
<213> Artificial

<220>
<223> mutant cap-leader

<400> 16
guauuaauac cagcuucaaa auauucc 27

<210> 17
<211> 27
<212> RNA
<213> Artificial

<220>
<223> mutant cap-leader

<400> 17
guauuaauac gagcuucaaa auauucc 27

<210> 18
<211> 27
<212> RNA
<213> Artificial

<220>
<223> mutant cap-leader

<400> 18
guauuaauac aagcuucaaa auauucc 27

<210> 19
<211> 27
<212> RNA
<213> Artificial

<220>
<223> mutant cap-leader

<400> 19
guauuaauac uagcuucaaa auauucc 27

<210> 20
<211> 27
<212> RNA
<213> Artificial

<220>
<223> mutant cap-leader

<400> 20
guauuaauac cagcuucaaa auauucc 27

<210> 21
<211> 27
<212> RNA
<213> Artificial

<220>
<223> mutant cap-leader

<400> 21
guauuaauag cagcuucaaa auauucc 27

<210> 22
<211> 27
<212> RNA
<213> Artificial

<220>
<223> mutant cap-leader

<400> 22
guauuaauaa cagcuucaaa auauucc 27

<210> 23
<211> 27

<212> RNA
<213> Artificial

<220>
<223> mutant cap-leader

<400> 23
guauuaauau cagcuucaaa auauucc 27


<210> 24
<211> 30
<212> RNA
<213> Artificial

<220>
<223> mutant cap-leader

<400> 24
guauuucagc uuuuuuuuuu uucaaaauau 30


<210> 25
<211> 30
<212> RNA
<213> Artificial

<220>
<223> mutant cap-leader

<400> 25
guauuuucag cuuuuuuuuu uucaaaauau 30


<210> 26
<211> 30
<212> RNA
<213> Artificial

<220>
<223> mutant cap-leader

<400> 26
guauuuuuca gcuuuuuuuu uucaaaauau 30


<210> 27
<211> 30
<212> RNA
<213> Artificial

<220>
<223> mutant cap-leader

<400> 27
guauuuuuc agcuuuuuu uucaaaauau 30

<210> 28
<211> 30
<212> RNA
<213> Artificial

<220>
<223> mutant cap leader

<400> 28
   guauuuuuuu cagcuuuuuu uucaaaauau 30

<210> 29
<211> 30
<212> RNA
<213> Artificial

<220>
<223> mutant cap leader

<400> 29
   guauuuuuuu ucagcuuuuu uucaaaauau 30

<210> 30
<211> 30
<212> RNA
<213> Artificial

<220>
<223> mutant cap-leader

<400> 30
   guauuuuuuu uucagcuuuu uucaaaauau 30

<210> 31
<211> 30
<212> RNA
<213> Artificial

<220>
<223> mutantcap-leader

<400> 31
   guauuuuuuu uuucagcuuu uucaaaauau 30

<210> 32
<211> 30

<212> RNA
<213> Artificial

<220>
<223> mutant cap-leader

<400> 32
guauuuuuuu uuuucagcuu uucaaaauau 30


<210> 33
<211> 30
<212> RNA
<213> Artificial

<220>
<223> mutant cap-leader

<400> 33
guauuuuuuu uuuuucagcu uucaaaauau 30


<210> 34
<211> 30
<212> RNA
<213> Artificial

<220>
<223> mutant cap-leader

<400> 34
guauuuuuuu uuuuuucagc uucaaaauau 30


<210> 35
<211> 18
<212> RNA
<213> Artificial

<220>
<223> retrieved mRNA

<400> 35
guauuaauac cagcaaaa 18


<210> 36
<211> 21
<212> RNA
<213> Artificial

<220>
<223> retrieved mRNA

<400> 36
guauuaauac cagcagcaaa a                                    21


<210> 37
<211> 18
<212> RNA
<213> Artificial

<220>
<223> Retrieved mRNA

<400> 37
guauuaauaa cagcaaaa                                    18


<210> 38
<211> 21
<212> RNA
<213> Artificial

<220>
<223> retrieved mRNA

<400> 38
guauuaauaa cagcagcaaa a                                    21


<210> 39
<211> 16
<212> RNA
<213> Artificial

<220>
<223> retrieved mRNA

<400> 39
guauuaauaa gcaaaa                                    16


<210> 40
<211> 17
<212> RNA
<213> Artificial

<220>
<223> retrieved mRNA

<400> 40
guauuaauaa cgcaaaa                                    17


<210> 41
<211> 15

<212> RNA
<213> Artificial

<220>
<223> retrieved mRNA

<400> 41
guauuaauag caaaa 15


<210> 42
<211> 18
<212> RNA
<213> Artificial

<220>
<223> retrieved mRNA

<400> 42
guauuaauau uagcaaaa 18


<210> 43
<211> 21
<212> RNA
<213> Artificial

<220>
<223> retrieved mRNA

<400> 43
guauuaauau cagcagcaaa a 21


<210> 44
<211> 18
<212> RNA
<213> Artificial

<220>
<223> retrieved mRNA

<400> 44
guauuaauac cagcaaaa 18


<210> 45
<211> 19
<212> RNA
<213> Artificial

<220>
<223> retrieved mRNA

<400> 45
guauuaauac gcagcaaaa 19

<210> 46
<211> 18
<212> RNA
<213> Artificial

<220>
<223> retrieved mRNA

<400> 46
guauuaauag cagcaaaa 18

<210> 47
<211> 19
<212> RNA
<213> Artificial

<220>
<223> retrieved mRNA

<400> 47
guauuaauau gcagcaaaa 19

<210> 48
<211> 18
<212> RNA
<213> Artificial

<220>
<223> retrieved mRNA

<400> 48
guauuaauag cagcaaaa 18

<210> 49
<211> 21
<212> RNA
<213> Artificial

<220>
<223> retrieved mRNA

<400> 49
guauuaauag cagcagcaaa a 21

<210> 50
<211> 18

<212> RNA
<213> Artificial

<220>
<223> retrieved mRNA

<400> 50
guauuaauac gagcaaaa 18

<210> 51
<211> 16
<212> RNA
<213> Artificial

<220>
<223> retrieved mRNA

<400> 51
guauuaauac gcaaaa 16

<210> 52
<211> 19
<212> RNA
<213> Artificial

<220>
<223> retrieved mRNA

<400> 52
guauuaauac gcagcaaaa 19

<210> 53
<211> 18
<212> RNA
<213> Artificial

<220>
<223> retrieved mRNA

<400> 53
guauuaauac aagcaaaa 18

<210> 54
<211> 17
<212> RNA
<213> Artificial

<220>
<223> retrieved mRNA

<400> 54
guauuaauac agcaaaa                                    17

<210> 55
<211> 18
<212> RNA
<213> Artificial

<220>
<223> retrieved mRNA

<400> 55
guauuaauac uagcaaaa                                   18

<210> 56
<211> 18
<212> RNA
<213> Artificial

<220>
<223> retrieved mRNA

<400> 56
guauuaauac cagcaaaa                                   18

<210> 57
<211> 21
<212> RNA
<213> Artificial

<220>
<223> retrieved mRNA

<400> 57
guauuaauac gagcagcaaa a                               21

<210> 58
<211> 18
<212> RNA
<213> Artificial

<220>
<223> retrieved mRNA

<400> 58
guauuaauag cagcaaaa                                   18

<210> 59
<211> 21

<212> RNA
<213> Artificial

<220>
<223> retrieved mRNA

<400> 59
guauuaauag cagcagcaaa a 21


<210> 60
<211> 18
<212> RNA
<213> Artificial

<220>
<223> retrieved mRNA

<400> 60
guauuaauaa cagcaaaa 18


<210> 61
<211> 18
<212> RNA
<213> Artificial

<220>
<223> retrieved mRNA

<400> 61
guauuaauau cagcaaaa 18


<210> 62
<211> 21
<212> RNA
<213> Artificial

<220>
<223> retrieved mRNA

<400> 62
guauuaauau cagcagcaaa a 21


<210> 63
<211> 18
<212> RNA
<213> Artificial

<220>
<223> retrieved mRNA

<400> 63
guauuaauac cagcaaaa                                                    18

<210> 64
<211> 18
<212> RNA
<213> Artificial

<220>
<223> retrieved mRNA

<400> 64
guauuaauac cagcaaaa                                                    18

<210> 65
<211> 21
<212> RNA
<213> Artificial

<220>
<223> retrieved mRNA

<400> 65
guauuaauac cagcagcaaa a                                                21

<210> 66
<211> 18
<212> RNA
<213> Artificial

<220>
<223> retrieved mRNA

<400> 66
guauuaauac aagcaaaa                                                    18

<210> 67
<211> 17
<212> RNA
<213> Artificial

<220>
<223> retrieved mRNA

<400> 67
guauuaauac agcaaaa                                                     17

<210> 68
<211> 18

<212> RNA
<213> Artificial

<220>
<223> retrieved mRNA

<400> 68
guauuuucag cagcaaaa 18


<210> 69
<211> 16
<212> RNA
<213> Artificial

<220>
<223> retrieved mRNA

<400> 69
guauuuuuca gcaaaa 16


<210> 70
<211> 19
<212> RNA
<213> Artificial

<220>
<223> retrieved mRNA

<400> 70
guauuuuuca gcagcaaaa 19


<210> 71
<211> 17
<212> RNA
<213> Artificial

<220>
<223> retrieved mRNA

<400> 71
guauuuuuuc agcaaaa 17


<210> 72
<211> 20
<212> RNA
<213> Artificial

<220>
<223> retrieved mRNA

<400> 72
   guauuuuuuc agcagcaaaa 20

<210> 73
<211> 18
<212> RNA
<213> Artificial

<220>
<223> retrieved mRNA

<400> 73
   guauuuuuuu cagcaaaa 18

<210> 74
<211> 19
<212> RNA
<213> Artificial

<220>
<223> retrieved mRNA

<400> 74
   guauuuuuuu ucagcaaaa 19

<210> 75
<211> 18
<212> RNA
<213> Artificial

<220>
<223> retrieved mRNA

<400> 75
auccuuuugc aagcaaaa 18

<210> 76
<211> 17
<212> RNA
<213> Artificial

<220>
<223> retrieved mRNA

<400> 76
aucuuuugca agcaaaa 17

<210> 77
<211> 21

<212> RNA
<213> Artificial

<220>
<223> retrieved mRNA

<400> 77
accggaggga gcaaagcaaa a 21


<210> 78
<211> 13
<212> RNA
<213> Artificial

<220>
<223> retrieved mRNA

<400> 78
aaguagagca aaa 13


<210> 79
<211> 16
<212> RNA
<213> Artificial

<220>
<223> retrieved mRNA

<400> 79
cugguugcgg gcaaaa 16


<210> 80
<211> 18
<212> RNA
<213> Artificial

<220>
<223> retrieved mRNA

<400> 80
cggaaggccu cggcaaaa 18


<210> 81
<211> 18
<212> RNA
<213> Artificial

<220>
<223> retrieved mRNA

<400> 81
agugucuccg cggcaaaa                                    18

<210> 82
<211> 19
<212> RNA
<213> Artificial

<220>
<223> retrieved mRNA

<400> 82
guucaucauc ccugcaaaa                                   19

<210> 83
<211> 16
<212> RNA
<213> Artificial

<220>
<223> retrieved mRNA

<400> 83
aaggcuugua gcaaaa                                      16

<210> 84
<211> 16
<212> RNA
<213> Artificial

<220>
<223> retrieved mRNA

<400> 84
aguguucgca gcaaaa                                      16

<210> 85
<211> 17
<212> RNA
<213> Artificial

<220>
<223> retrieved mRNA

<400> 85
aacaaacuuc agcaaaa                                     17

<210> 86
<211> 14

<212> DNA
<213> Artificial

<220>
<223> primer

<400> 86
cccggatccg gggg 14


<210> 87
<211> 25
<212> DNA
<213> Artificial

<220>
<223> primer

<400> 87
cccggatccg ggggacactt ctggt 25


<210> 88
<211> 23
<212> DNA
<213> Artificial

<220>
<223> primer

<400> 88
cccggatccg gggggtatta ata 23


<210> 89
<211> 19
<212> DNA
<213> Artificial

<220>
<223> primer

<400> 89
cccggatccg gggggtatt 19


<210> 90
<211> 25
<212> DNA
<213> Artificial

<220>
<223> primer

<400> 90
cccgaattcg agtctccagc cggtc 25

<210> 91
<211> 25
<212> DNA
<213> Artificial

<220>
<223> primer

<400> 91
cccgaattcc gcctggtcca ttctg 25

<210> 92
<211> 28
<212> DNA
<213> Artificial

<220>
<223> primer

<400> 92
cccgaattcg agtcagacca gccgttgc 28

<210> 93
<211> 28
<212> DNA
<213> Artificial

<220>
<223> primer

<400> 93
cccgaattcg cttggcgcca gattcgcc 28

<210> 94
<211> 53
<212> DNA
<213> Artificial

<220>
<223> primer

<400> 94
cccggatcct aatacgactc actatagtat taataccatt ttcaaaatat tcc 53

<210> 95
<211> 53

<212> DNA
<213> Artificial

<220>
<223> primer

<400> 95
cccggatcct aatacgactc actatagtat taataacagt ttcaaaatat tcc 53


<210> 96
<211> 53
<212> DNA
<213> Artificial

<220>
<223> primer

<400> 96
cccggatcct aatacgactc actatagtat taatagcagc ttcaaaatat tcc 53


<210> 97
<211> 48
<212> DNA
<213> Artificial

<220>
<223> primer

<400> 97
cccggatcct aatacgactc actatagtat taatatcagc aaaagcag 48


<210> 98
<211> 34
<212> DNA
<213> Artificial

<220>
<223> primer


<220>
<221> misc_feature
<222> (18)..(18)
<223> n is a, c, g, or t

<400> 98
cactatagta ttaatacnat tttcaaaata ttcc 34


<210> 99
<211> 34

<212> DNA
<213> Artificial

<220>
<223> primer


<220>
<221> misc_feature
<222> (17)..(17)
<223> n is a, c, g, or t

<400> 99
cactatagta ttaatancag cttcaaaata ttcc 34


<210> 100
<211> 34
<212> DNA
<213> Artificial

<220>
<223> primer


<220>
<221> misc_feature
<222> (18)..(18)
<223> n is a, c, g, or t

<400> 100
cactatagta ttaatacnag cttcaaaata ttcc 34


<210> 101
<211> 48
<212> DNA
<213> Artificial

<220>
<223> primer

<400> 101
cgactcacta tagtatttca gctttttttt ttttcaaaat attccaat 48


<210> 102
<211> 48
<212> DNA
<213> Artificial

<220>
<223> primer

<400> 102
cgactcacta tagtattttc agcttttttt ttttcaaaat attccaat 48

<210> 103
<211> 48
<212> DNA
<213> Artificial

<220>
<223> primer

<400> 103
cgactcacta tagtattttt cagctttttt ttttcaaaat attccaat 48

<210> 104
<211> 48
<212> DNA
<213> Artificial

<220>
<223> primer

<400> 104
cgactcacta tagtattttt tcagcttttt ttttcaaaat attccaat 48

<210> 105
<211> 48
<212> DNA
<213> Artificial

<220>
<223> primer

<400> 105
cgactcacta tagtattttt ttcagctttt ttttcaaaat attccaat 48

<210> 106
<211> 48
<212> DNA
<213> Artificial

<220>
<223> primer

<400> 106
cgactcacta tagtattttt tttcagcttt ttttcaaaat attccaat 48

<210> 107
<211> 48

<212> DNA
<213> Artificial

<220>
<223> primer

<400> 107
cgactcacta tagtattttt ttttcagctt ttttcaaaat attccaat 48


<210> 108
<211> 48
<212> DNA
<213> Artificial

<220>
<223> primer

<400> 108
cgactcacta tagtattttt tttttcagct ttttcaaaat attccaat 48


<210> 109
<211> 48
<212> DNA
<213> Artificial

<220>
<223> primer

<400> 109
cgactcacta tagtattttt ttttttcagc ttttcaaaat attccaat 48


<210> 110
<211> 48
<212> DNA
<213> Artificial

<220>
<223> primer

<400> 110
cgactcacta tagtattttt tttttttcag ctttcaaaat attccaat 48


<210> 111
<211> 48
<212> DNA
<213> Artificial

<220>
<223> primer

<400> 111
cgactcacta tagtattttt tttttttca gcttcaaaat attccaat 48

<210> 112
<211> 36
<212> DNA
<213> Artificial

<220>
<223> primer

<400> 112
cccggatcct aatacgactc actatagtat taatac 36

<210> 113
<211> 31
<212> DNA
<213> Artificial

<220>
<223> primer

<400> 113
cccggatcct aatacgactc actatagtat t 31

<210> 114
<211> 32
<212> DNA
<213> Artificial

<220>
<223> primer

<400> 114
cccggatcct aatacgactc actatagtat tt 32

<210> 115
<211> 28
<212> DNA
<213> Artificial

<220>
<223> primer

<400> 115
cccgaattcg aagagtacga attacgcg 28

SEQUENCE LISTING

<110>  Wageningen Universiteit

<120>  Influenza cap-leader sequence

<130>  P83864EP00

<160>  115

<170>  PatentIn version 3.3

<210>  1
<211>  12
<212>  RNA
<213>  Artificial

<220>
<223>  consensus (n=6)


<220>
<221>  modified_base
<222>  (1)..(1)
<223>  m7g

<220>
<221>  misc_feature
<222>  (2)..(7)
<223>  n is a, c, g, or u

<400>  1
gnnnnnndwa gc                                                          12


<210>  2
<211>  13
<212>  RNA
<213>  Artificial

<220>
<223>  consensus (n=7)


<220>
<221>  modified_base
<222>  (1)..(1)
<223>  m7g

<220>
<221>  misc_feature
<222>  (2)..(8)
<223>  n is a, c, g, or u

<400>  2
gnnnnnnndw agc                                                         13


<210>  3
<211>  14
<212>  RNA
<213>  Artificial

<220>
<223>  consensus (n=8)


<220>
<221>  modified_base

<222>  (1)..(1)
<223>  m7g

<220>
<221>  misc_feature
<222>  (2)..(9)
<223>  n is a, c, g, or u

<400>  3
   gnnnnnnnnd wagc                                                          14


<210>  4
<211>  15
<212>  RNA
<213>  Artificial

<220>
<223>  consensus (n=9)


<220>
<221>  modified_base
<222>  (1)..(1)
<223>  m7g

<220>
<221>  misc_feature
<222>  (2)..(10)
<223>  n is a, c, g, or u

<400>  4
gnnnnnnnnn dwagc                                                        15


<210>  5
<211>  16
<212>  RNA
<213>  Artificial

<220>
<223>  consensus (n=10)


<220>
<221>  modified_base
<222>  (1)..(1)
<223>  m7g

<220>
<221>  misc_feature
<222>  (2)..(11)
<223>  n is a, c, g, or u

<400>  5
gnnnnnnnnn ndwagc                                                       16


<210>  6
<211>  12
<212>  RNA
<213>  Influenza A virus

<400>  6
uucgcuucug cu                                                           12


<210>  7
<211>  12

<212>  RNA
<213>  Influenza B virus

<400>  7
ccugcuuuug cu                                                          12


<210>  8
<211>  27
<212>  RNA
<213>  Alfalfa mosaic virus

<400>  8
guauuaauac cauuuucaaa auauucc                                          27


<210>  9
<211>  27
<212>  RNA
<213>  Artificial

<220>
<223>  mutant cap-leader

<400>  9
guauuaauaa caguuucaaa auauucc                                          27


<210>  10
<211>  27
<212>  RNA
<213>  Artificial

<220>
<223>  mutant cap-leader

<400>  10
guauuaauag cagcuucaaa auauucc                                          27


<210>  11
<211>  25
<212>  RNA
<213>  Artificial

<220>
<223>  mutant cap-leader

<400>  11
guauuaauau cagcaaaagc agggu                                            25


<210>  12
<211>  27
<212>  RNA
<213>  Artificial

<220>
<223>  mutant cap-leader

<400>  12
guauuaauac cauuuucaaa auauucc                                          27


<210>  13
<211>  27
<212>  RNA
<213>  Artificial

<220>
<223>  mutant cap-leader

<400>  13
guauuaauac gauuuucaaa auauucc                                          27


<210>  14
<211>  27
<212>  RNA
<213>  Artificial

<220>
<223>  mutant cap-leader

<400>  14
guauuaauac aauuuucaaa auauucc                                          27


<210>  15
<211>  27
<212>  RNA
<213>  Artificial

<220>
<223>  mutant cap-leader

<400>  15
guauuaauac uauuuucaaa auauucc                                          27


<210>  16
<211>  27
<212>  RNA
<213>  Artificial

<220>
<223>  mutant cap-leader

<400>  16
guauuaauac cagcuucaaa auauucc                                          27


<210>  17
<211>  27
<212>  RNA
<213>  Artificial

<220>
<223>  mutant cap-leader

<400>  17
guauuaauac gagcuucaaa auauucc                                          27


<210>  18
<211>  27
<212>  RNA
<213>  Artificial

<220>
<223>  mutant cap-leader

<400>  18
guauuaauac aagcuucaaa auauucc                                          27


<210>  19
<211>  27

<212>  RNA
<213>  Artificial

<220>
<223>  mutant cap-leader

<400>  19
guauuaauac uagcuucaaa auauucc                                          27


<210>  20
<211>  27
<212>  RNA
<213>  Artificial

<220>
<223>  mutant cap-leader

<400>  20
guauuaauac cagcuucaaa auauucc                                          27


<210>  21
<211>  27
<212>  RNA
<213>  Artificial

<220>
<223>  mutant cap-leader

<400>  21
guauuaauag cagcuucaaa auauucc                                          27


<210>  22
<211>  27
<212>  RNA
<213>  Artificial

<220>
<223>  mutant cap-leader

<400>  22
guauuaauaa cagcuucaaa auauucc                                          27


<210>  23
<211>  27
<212>  RNA
<213>  Artificial

<220>
<223>  mutant cap-leader

<400>  23
guauuaauau cagcuucaaa auauucc                                          27


<210>  24
<211>  30
<212>  RNA
<213>  Artificial

<220>
<223>  mutant cap-leader

<400>  24
guauuucagc uuuuuuuuuu uucaaaauau                                       30

<210>  25
<211>  30
<212>  RNA
<213>  Artificial

<220>
<223>  mutant cap-leader

<400>  25
guauuuucag cuuuuuuuuu uucaaaauau                                          30


<210>  26
<211>  30
<212>  RNA
<213>  Artificial

<220>
<223>  mutant cap-leader

<400>  26
guauuuuuca gcuuuuuuuu uucaaaauau                                          30


<210>  27
<211>  30
<212>  RNA
<213>  Artificial

<220>
<223>  mutant cap-leader

<400>  27
guauuuuuuc agcuuuuuuu uucaaaauau                                          30


<210>  28
<211>  30
<212>  RNA
<213>  Artificial

<220>
<223>  mutant cap leader

<400>  28
guauuuuuuu cagcuuuuuu uucaaaauau                                          30


<210>  29
<211>  30
<212>  RNA
<213>  Artificial

<220>
<223>  mutant cap leader

<400>  29
guauuuuuuu ucagcuuuuu uucaaaauau                                          30


<210>  30
<211>  30
<212>  RNA
<213>  Artificial

<220>
<223>  mutant cap-leader

<400>  30
guauuuuuuu uucagcuuuu uucaaaauau                                        30


<210>  31
<211>  30
<212>  RNA
<213>  Artificial

<220>
<223>  mutantcap-leader

<400>  31
guauuuuuuu uuucagcuuu uucaaaauau                                        30


<210>  32
<211>  30
<212>  RNA
<213>  Artificial

<220>
<223>  mutant cap-leader

<400>  32
guauuuuuuu uuuucagcuu uucaaaauau                                        30


<210>  33
<211>  30
<212>  RNA
<213>  Artificial

<220>
<223>  mutant cap-leader

<400>  33
guauuuuuuu uuuuucagcu uucaaaauau                                        30


<210>  34
<211>  30
<212>  RNA
<213>  Artificial

<220>
<223>  mutant cap-leader

<400>  34
guauuuuuuu uuuuuucagc uucaaaauau                                        30


<210>  35
<211>  18
<212>  RNA
<213>  Artificial

<220>
<223>  retrieved mRNA

<400>  35
guauuaauac cagcaaaa                                                     18


<210>  36
<211>  21
<212>  RNA
<213>  Artificial

<220>
<223>  retrieved mRNA

<400>  36
guauuaauac cagcagcaaa a                                                21


<210>  37
<211>  18
<212>  RNA
<213>  Artificial

<220>
<223>  Retrieved mRNA

<400>  37
guauuaauaa cagcaaaa                                                    18


<210>  38
<211>  21
<212>  RNA
<213>  Artificial

<220>
<223>  retrieved mRNA

<400>  38
guauuaauaa cagcagcaaa a                                                21


<210>  39
<211>  16
<212>  RNA
<213>  Artificial

<220>
<223>  retrieved mRNA

<400>  39
guauuaauaa gcaaaa                                                      16


<210>  40
<211>  17
<212>  RNA
<213>  Artificial

<220>
<223>  retrieved mRNA

<400>  40
guauuaauaa cgcaaaa                                                     17


<210>  41
<211>  15
<212>  RNA
<213>  Artificial

<220>
<223>  retrieved mRNA

<400>  41
guauuaauag caaaa                                                       15


<210>  42
<211>  18

<212>  RNA
<213>  Artificial

<220>
<223>  retrieved mRNA

<400>  42
guauuaauau uagcaaaa                                                    18


<210>  43
<211>  21
<212>  RNA
<213>  Artificial

<220>
<223>  retrieved mRNA

<400>  43
guauuaauau cagcagcaaa a                                                21


<210>  44
<211>  18
<212>  RNA
<213>  Artificial

<220>
<223>  retrieved mRNA

<400>  44
guauuaauac cagcaaaa                                                    18


<210>  45
<211>  19
<212>  RNA
<213>  Artificial

<220>
<223>  retrieved mRNA

<400>  45
guauuaauac gcagcaaaa                                                   19


<210>  46
<211>  18
<212>  RNA
<213>  Artificial

<220>
<223>  retrieved mRNA

<400>  46
guauuaauag cagcaaaa                                                    18


<210>  47
<211>  19
<212>  RNA
<213>  Artificial

<220>
<223>  retrieved mRNA

<400>  47
guauuaauau gcagcaaaa                                                   19

<210>  48
<211>  18
<212>  RNA
<213>  Artificial

<220>
<223>  retrieved mRNA

<400>  48
guauuaauag cagcaaaa                                                    18


<210>  49
<211>  21
<212>  RNA
<213>  Artificial

<220>
<223>  retrieved mRNA

<400>  49
guauuaauag cagcagcaaa a                                                21


<210>  50
<211>  18
<212>  RNA
<213>  Artificial

<220>
<223>  retrieved mRNA

<400>  50
guauuaauac gagcaaaa                                                    18


<210>  51
<211>  16
<212>  RNA
<213>  Artificial

<220>
<223>  retrieved mRNA

<400>  51
guauuaauac gcaaaa                                                      16


<210>  52
<211>  19
<212>  RNA
<213>  Artificial

<220>
<223>  retrieved mRNA

<400>  52
guauuaauac gcagcaaaa                                                   19


<210>  53
<211>  18
<212>  RNA
<213>  Artificial

<220>
<223>  retrieved mRNA

<400>  53
guauuaauac aagcaaaa                                                    18


<210>  54
<211>  17
<212>  RNA
<213>  Artificial

<220>
<223>  retrieved mRNA

<400>  54
guauuaauac agcaaaa                                                     17


<210>  55
<211>  18
<212>  RNA
<213>  Artificial

<220>
<223>  retrieved mRNA

<400>  55
guauuaauac uagcaaaa                                                    18


<210>  56
<211>  18
<212>  RNA
<213>  Artificial

<220>
<223>  retrieved mRNA

<400>  56
guauuaauac cagcaaaa                                                    18


<210>  57
<211>  21
<212>  RNA
<213>  Artificial

<220>
<223>  retrieved mRNA

<400>  57
guauuaauac gagcagcaaa a                                                21


<210>  58
<211>  18
<212>  RNA
<213>  Artificial

<220>
<223>  retrieved mRNA

<400>  58
guauuaauag cagcaaaa                                                    18


<210>  59
<211>  21
<212>  RNA
<213>  Artificial

<220>
<223>  retrieved mRNA

<400>  59
guauuaauag cagcagcaaa a                                                21


<210>  60
<211>  18
<212>  RNA
<213>  Artificial

<220>
<223>  retrieved mRNA

<400>  60
guauuaauaa cagcaaaa                                                    18


<210>  61
<211>  18
<212>  RNA
<213>  Artificial

<220>
<223>  retrieved mRNA

<400>  61
guauuaauau cagcaaaa                                                    18


<210>  62
<211>  21
<212>  RNA
<213>  Artificial

<220>
<223>  retrieved mRNA

<400>  62
guauuaauau cagcagcaaa a                                                21


<210>  63
<211>  18
<212>  RNA
<213>  Artificial

<220>
<223>  retrieved mRNA

<400>  63
guauuaauac cagcaaaa                                                    18


<210>  64
<211>  18
<212>  RNA
<213>  Artificial

<220>
<223>  retrieved mRNA

<400>  64
guauuaauac cagcaaaa                                                    18


<210>  65
<211>  21

<212>  RNA
<213>  Artificial

<220>
<223>  retrieved mRNA

<400>  65
guauuaauac cagcagcaaa a                                                21


<210>  66
<211>  18
<212>  RNA
<213>  Artificial

<220>
<223>  retrieved mRNA

<400>  66
guauuaauac aagcaaaa                                                    18


<210>  67
<211>  17
<212>  RNA
<213>  Artificial

<220>
<223>  retrieved mRNA

<400>  67
guauuaauac agcaaaa                                                     17


<210>  68
<211>  18
<212>  RNA
<213>  Artificial

<220>
<223>  retrieved mRNA

<400>  68
guauuuucag cagcaaaa                                                    18


<210>  69
<211>  16
<212>  RNA
<213>  Artificial

<220>
<223>  retrieved mRNA

<400>  69
guauuuuuca gcaaaa                                                      16


<210>  70
<211>  19
<212>  RNA
<213>  Artificial

<220>
<223>  retrieved mRNA

<400>  70
guauuuuuca gcagcaaaa                                                   19

<210>  71
<211>  17
<212>  RNA
<213>  Artificial

<220>
<223>  retrieved mRNA

<400>  71
guauuuuuc agcaaaa                                                         17


<210>  72
<211>  20
<212>  RNA
<213>  Artificial

<220>
<223>  retrieved mRNA

<400>  72
guauuuuuc agcagcaaaa                                                      20


<210>  73
<211>  18
<212>  RNA
<213>  Artificial

<220>
<223>  retrieved mRNA

<400>  73
guauuuuuu cagcaaaa                                                        18


<210>  74
<211>  19
<212>  RNA
<213>  Artificial

<220>
<223>  retrieved mRNA

<400>  74
guauuuuuu ucagcaaaa                                                       19


<210>  75
<211>  18
<212>  RNA
<213>  Artificial

<220>
<223>  retrieved mRNA

<400>  75
auccuuugc aagcaaaa                                                        18


<210>  76
<211>  17
<212>  RNA
<213>  Artificial

<220>
<223>  retrieved mRNA

<400>  76
aucuuuugca agcaaaa                                                      17


<210>  77
<211>  21
<212>  RNA
<213>  Artificial

<220>
<223>  retrieved mRNA

<400>  77
accggaggga gcaaagcaaa a                                                 21


<210>  78
<211>  13
<212>  RNA
<213>  Artificial

<220>
<223>  retrieved mRNA

<400>  78
aaguagagca aaa                                                          13


<210>  79
<211>  16
<212>  RNA
<213>  Artificial

<220>
<223>  retrieved mRNA

<400>  79
cugguugcgg gcaaaa                                                       16


<210>  80
<211>  18
<212>  RNA
<213>  Artificial

<220>
<223>  retrieved mRNA

<400>  80
cggaaggccu cggcaaaa                                                     18


<210>  81
<211>  18
<212>  RNA
<213>  Artificial

<220>
<223>  retrieved mRNA

<400>  81
agugucuccg cggcaaaa                                                     18


<210>  82
<211>  19
<212>  RNA
<213>  Artificial

<220>
<223>  retrieved mRNA

<400>  82
guucaucauc ccugcaaaa                                                    19


<210>  83
<211>  16
<212>  RNA
<213>  Artificial

<220>
<223>  retrieved mRNA

<400>  83
aaggcuugua gcaaaa                                                       16


<210>  84
<211>  16
<212>  RNA
<213>  Artificial

<220>
<223>  retrieved mRNA

<400>  84
aguguucgca gcaaaa                                                       16


<210>  85
<211>  17
<212>  RNA
<213>  Artificial

<220>
<223>  retrieved mRNA

<400>  85
aacaaacuuc agcaaaa                                                      17


<210>  86
<211>  14
<212>  DNA
<213>  Artificial

<220>
<223>  primer

<400>  86
cccggatccg gggg                                                         14


<210>  87
<211>  25
<212>  DNA
<213>  Artificial

<220>
<223>  primer

<400>  87
   cccggatccg ggggacactt ctggt                                              25


<210>  88
<211>  23

<212>  DNA
<213>  Artificial

<220>
<223>  primer

<400>  88
cccggatccg gggggtatta ata                                              23


<210>  89
<211>  19
<212>  DNA
<213>  Artificial

<220>
<223>  primer

<400>  89
cccggatccg gggggtatt                                                   19


<210>  90
<211>  25
<212>  DNA
<213>  Artificial

<220>
<223>  primer

<400>  90
cccgaattcg agtctccagc cggtc                                            25


<210>  91
<211>  25
<212>  DNA
<213>  Artificial

<220>
<223>  primer

<400>  91
cccgaattcc gcctggtcca ttctg                                            25


<210>  92
<211>  28
<212>  DNA
<213>  Artificial

<220>
<223>  primer

<400>  92
cccgaattcg agtcagacca gccgttgc                                         28


<210>  93
<211>  28
<212>  DNA
<213>  Artificial

<220>
<223>  primer

<400>  93
cccgaattcg cttggcgcca gattcgcc                                         28

<210>  94
<211>  53
<212>  DNA
<213>  Artificial

<220>
<223>  primer

<400>  94
cccggatcct aatacgactc actatagtat taataccatt ttcaaaatat tcc          53


<210>  95
<211>  53
<212>  DNA
<213>  Artificial

<220>
<223>  primer

<400>  95
cccggatcct aatacgactc actatagtat taataacagt ttcaaaatat tcc          53


<210>  96
<211>  53
<212>  DNA
<213>  Artificial

<220>
<223>  primer

<400>  96
cccggatcct aatacgactc actatagtat taatagcagc ttcaaaatat tcc          53


<210>  97
<211>  48
<212>  DNA
<213>  Artificial

<220>
<223>  primer

<400>  97
cccggatcct aatacgactc actatagtat taatatcagc aaaagcag                48


<210>  98
<211>  34
<212>  DNA
<213>  Artificial

<220>
<223>  primer


<220>
<221>  misc_feature
<222>  (18)..(18)
<223>  n is a, c, g, or t

<400>  98
cactatagta ttaatacnat tttcaaaata ttcc                               34


<210>  99
<211>  34

<212>  DNA
<213>  Artificial

<220>
<223>  primer


<220>
<221>  misc_feature
<222>  (17)..(17)
<223>  n is a, c, g, or t

<400>  99
cactatagta ttaatancag cttcaaaata ttcc                                34


<210>  100
<211>  34
<212>  DNA
<213>  Artificial

<220>
<223>  primer


<220>
<221>  misc_feature
<222>  (18)..(18)
<223>  n is a, c, g, or t

<400>  100
cactatagta ttaatacnag cttcaaaata ttcc                                34


<210>  101
<211>  48
<212>  DNA
<213>  Artificial

<220>
<223>  primer

<400>  101
cgactcacta tagtatttca gctttttttt ttttcaaaat attccaat                  48


<210>  102
<211>  48
<212>  DNA
<213>  Artificial

<220>
<223>  primer

<400>  102
cgactcacta tagtattttc agcttttttt ttttcaaaat attccaat                  48


<210>  103
<211>  48
<212>  DNA
<213>  Artificial

<220>
<223>  primer

<400>  103
cgactcacta tagtattttt cagctttttt ttttcaaaat attccaat                  48

<210>  104
<211>  48
<212>  DNA
<213>  Artificial

<220>
<223>  primer

<400>  104
cgactcacta tagtattttt tcagcttttt ttttcaaaat attccaat                    48


<210>  105
<211>  48
<212>  DNA
<213>  Artificial

<220>
<223>  primer

<400>  105
cgactcacta tagtattttt ttcagctttt ttttcaaaat attccaat                    48


<210>  106
<211>  48
<212>  DNA
<213>  Artificial

<220>
<223>  primer

<400>  106
cgactcacta tagtattttt tttcagcttt ttttcaaaat attccaat                    48


<210>  107
<211>  48
<212>  DNA
<213>  Artificial

<220>
<223>  primer

<400>  107
cgactcacta tagtattttt ttttcagctt ttttcaaaat attccaat                    48


<210>  108
<211>  48
<212>  DNA
<213>  Artificial

<220>
<223>  primer

<400>  108
cgactcacta tagtattttt tttttcagct ttttcaaaat attccaat                    48


<210>  109
<211>  48
<212>  DNA
<213>  Artificial

<220>
<223>  primer

<400>  109
cgactcacta tagtattttt ttttttcagc ttttcaaaat attccaat                    48


<210>  110
<211>  48
<212>  DNA
<213>  Artificial

<220>
<223>  primer

<400>  110
cgactcacta tagtattttt tttttttcag ctttcaaaat attccaat                    48


<210>  111
<211>  48
<212>  DNA
<213>  Artificial

<220>
<223>  primer

<400>  111
cgactcacta tagtattttt ttttttttca gcttcaaaat attccaat                    48


<210>  112
<211>  36
<212>  DNA
<213>  Artificial

<220>
<223>  primer

<400>  112
cccggatcct aatacgactc actatagtat taatac                                 36


<210>  113
<211>  31
<212>  DNA
<213>  Artificial

<220>
<223>  primer

<400>  113
cccggatcct aatacgactc actatagtat t                                      31


<210>  114
<211>  32
<212>  DNA
<213>  Artificial

<220>
<223>  primer

<400>  114
cccggatcct aatacgactc actatagtat tt                                     32


<210>  115
<211>  28
<212>  DNA
<213>  Artificial

<220>
<223>  primer

<400>  115
cccgaattcg aagagtacga attacgcg                                          28

## Description

This invention relates to the field of viral genome transcription, more particular to the field of cap-leader sequences involved in initiation of Influenza genome transcription, more especially to the field of strong consensus Influenza cap-leader sequences. Further the invention relates to novel therapeutic substances for inhibiting viral infections.

"Cap-snatching" is a special mechanism of viral transcription initiation-found among the segmented (-)ssRNA viruses and first discovered for Influenza A (Bouloy et al., 1978; Plotch et al., 1979; Krug et al., 1979; Caton and Robertson, 1980; Dhar et al., 1980; Beaton and Krug, 1981; Plotch et al., 1981). During this mechanism, the viral transcriptase cleaves m⁷G-capped RNA leader sequences from host mRNAs to prime transcription of the viral genome. Nascent viral transcripts thereby are provided a 5' cap structure and a non-viral leader sequence, which allows them to be distinguished from (anti)genomic viral RNA molecules. Cap-snatching has later been reported for all segmented (-)ssRNA viruses irrespective of their host (animal, human or plants) (Kormelink et al., 1992; Huiet et al., 1993; Jin and Elliott, 1993a and b; Garcin et al., 1995; Duijsings et al., 2001), with the exception of Ophioviruses.

Whereas most of these viruses replicate in the cytoplasm and use the existing pool of host mRNAs as substrate, Influenza A virus replicates in the nucleus of the infected cell where it uses nascently synthesized transcripts from the RNA polymerase II complex (Fodor et al., 2000; Engelhardt et al., 2005; Chan et al., 2006). During the last two decades, the use of various *in vitro* systems (Bouloy et al., 1978; Plotch et al., 1979; Hagen et al., 1994) and the establishment of reverse genetics systems (Pleschka et al., 1996; Fodor et al., 1999; Neumann et al., 1999) have advanced the insight into cis- and transacting factors involved in cap-snatching. However, still many steps during this process remain an enigma or a matter of debate.

The Influenza A polymerase complex consists of three viral proteins, PB1, PB2 and PA, which are all required for viral mRNA synthesis. Whilst the role of PA is still unknown, PB2 binds to the 5' cap structure of cellular mRNAs (Licheng et al., 1995), which activates its endonuclease domain to cleave at a site 10-14 nucleotides downstream the 5' cap structure. During mRNA chain elongation, PB1 catalyses nucleotide addition (Braam et al, 1983). Whereas several reports suggested that the presence of the 5'terminal end of the viral RNA template (vRNA) is required and sufficient for activation of cap-dependent endonuclease activity (Hagen et al., 1994; Cianci et al., 1995), others have suggested that endonuclease activation requires a specific base pairing between several residues of the 5'- and 3'-terminal end of the vRNA template (Li et al., 2001).

Endonuclease cleavage generally takes place around 15 nt from the 5' cap structure, though variation in leader length between 10 to 20 nucleotides is observed. Exceptions are reported for Dugbe nairovirus (Bunyaviridae) and Tacaribe arenavirus (Arenaviridae) which use relatively short capped RNA leaders (1-4 resp. 5-16 nts). For several viruses, sequence analyses of viral mRNAs have shown a nucleotide preference at the 3' end of the non-viral leader, which is assumed to reflect a sequence preference for endonuclease cleavage. For Influenza A virus a preference for purine residues has been proposed (Plotch et al., 1981; Beaton and Krug, 1981), and only those capped RNA leaders harboring a 3'-terminal CA dinucleotide are effectively used as primers (Beaton and Krug, 1981; Rao et al., 2003). For Bunyamwera and Dugbe virus, both members of the Bunyaviridae, a strong preference for cleavage after a U or C residue respectively has been postulated (Jin and Elliott, 1993a and b). In all these cases, however, most sequences resulted from only a limited amount of mRNAs produced *in vivo*, and the host mRNAs from which the capped leader sequences originated remained unknown.

Hence, it can not be ruled out that for all segmented (-)ssRNA viruses, the capped leader may as well have been cleaved further downstream the assumed 3' end, e.g. 1-2 nucleotides, to provide several residues which assist in the alignment of capped RNA leader molecules along the viral template by virtue of base pairing. This idea is not only tempting but also supported by several lines of evidence presented in literature.

I*n vitro* studies on Influenza A virus suggest base pair interactions may contribute to the alignment of capped RNA leader molecules to the viral RNA template (Plotch et al., 1981; Hagen et al., 1994; Chung et al., 1994), although some conflicting data exists (Krug et al., 1980; Rao et al., 2003).

A "prime-and-realign" mechanism has been proposed for Hantaan virus genome transcription to explain the presence of repetitive sequences within the leader sequence of viral transcripts. During this process re-alignment of a capped leader sequence to the viral RNA template relies on base pairing interaction. Since its first description (Garcin et al, 1995), the presence of repeated sequences in viral leader sequences of viruses from the *Bunyaviridae* have been attributed, partly in retrospective, to the occurrence of "prime-and-realign" (Bishop et al. 1983; Bouloy et al., 1990; Simons and Pettersson, 1991; Jin and Elliott, 1993b; Duijsings et al., 2001; van Knippenberg et al., 2002).

*In vivo* transcription studies on TSWV during a mixed infection with Alfalfa mosaic virus (AMV) of Nicotiana benthamiana, and on TSWV-infected N. tabacum plants transgenically expressing the AMV replicase p1 and p2 proteins co-inoculated with (mutant) AMV-RNA3 (AMV3) constructs, have demonstrated a requirement for base complementarity of AMV-derived capped RNA leaders to the ultimate or penultimate 3' residue of the TSWV RNA template (Duijsings et al., 1999 and 2001). Similar results have been obtained using an *in vitro* transcription system based on purified TSWV particles supplemented with Rabbit Reticulocyte Lysate (RRL) (van Knippenberg et al., 2002). Furthermore, pair-wise competition of several (mutant) cap donors have shown that TSWV prefers capped leaders with multiple base complementarity to the viral template RNA, even when offered at relative low amounts compared to others with less base complementarity (van Knippenberg et al., 2005).

In light of the studies on TSWV, the highly conserved nature of cap-snatching, and the absence of clear quantitative (competition) data on the use of different cap donors during Influenza A virus transcription, the apparent preference of Influenza for endonuclease cleavage at purine residues and for capped RNA leaders harboring a 3'-terminal CA dinucleotide can be questioned.

It is generally accepted that infections with Influenza A virus are a serious threat to mankind. Also reason for serious concern is the observation that Influenza viruses can obtain (via mutations) the capacity to spread between different host species as observed for the H5N1 virus (poultry and human). Accordingly it should be feared that H5N1 can mutate into a strain capable of efficient human-to-human transmission.

Current options to limit the clinical impact of influenza virus infections include vaccination and the use of antiviral drugs. Since the current manufacturing capacity for vaccines is insufficient to cope with the demand during a pandemic, the availability of antiviral drugs may help to contain the emerging pandemic virus at its emergence.

The efficacy of the two currently available classes of antiviral drugs, neuraminidase inhibitors (e.g. Tamiflu) and ion channel blockers, is limited and heavily dependent of a very early start of treatment. In addition, several cases of resistance have already been reported.

Thus there is a growing need for additional alternatives to combat Influenza viral infections .A better understanding of the requirements for cap-snatching during Influenza virus transcription will provide insight in possible ways to inhibit transcription, subsequently leading to new antiviral strategies that can be used in drug development.

### Summary of the invention

The inventors have now further specified the requirements for the cap-leader sequence necessary for optimal initiation of the viral genome transcription of (-)ssRNA viruses. The current invention provides a cap-leader sequence comprising at least a single up to three base complementarity to the 3' residues of the viral RNA. This cap leader preferably has the consensus sequence ^{7m}G (N)₆₋₁₀-(A/U/G)-A/U-AGC. Also part of the invention is a modification of the cap-leader sequence to avoid elongation and/or endonuclease cleavage by the viral polymerase.

Said modification preferably is a modification of the 3'-OH terminated cap-leader into a 3'-phosphate. Alternatively said modification is a modification of the phosphodiester bonds within the cap-leader to avoid cleavage by the viral endonuclease (and other host nucleases) to render a cap-leader sequence capable of becoming elongated. Said modification preferably is a replacement of the phosphodiester bonds within the cap-leader into phosphorothioate bonds or phosphorodiamidate bonds. Preferably at least the phosphodiester bond between the A, G, or C at the 3' terminus is replaced. Most preferably the complete molecule has phosphorothiate or phosphorodiamidate bonds. Alternatively said modification is a replacement of one or more ribose ring(s) (or when a DNA consensus sequence is used of the dideoxyribose) within the cap-leader with one or more morpholino ring(s).

The invention further provides for a method to inhibit genome transcription of a (-)ssRNA virus in a cell by providing said cell with a modified cap-leader which has at least three bases complementary to the 3'ultimate residues of the viral template wherein the modifications is chosen from the modifications listed above. Preferably said virus is Influenza (A, B or C) and the 3'-ultimate residues have the sequence UCG.

Further, the invention pertains to the use of the above describe sequence and method to inhibit or treat a viral infection wherein the viral infection preferably is an Influenza infection.

### Legends to the figures

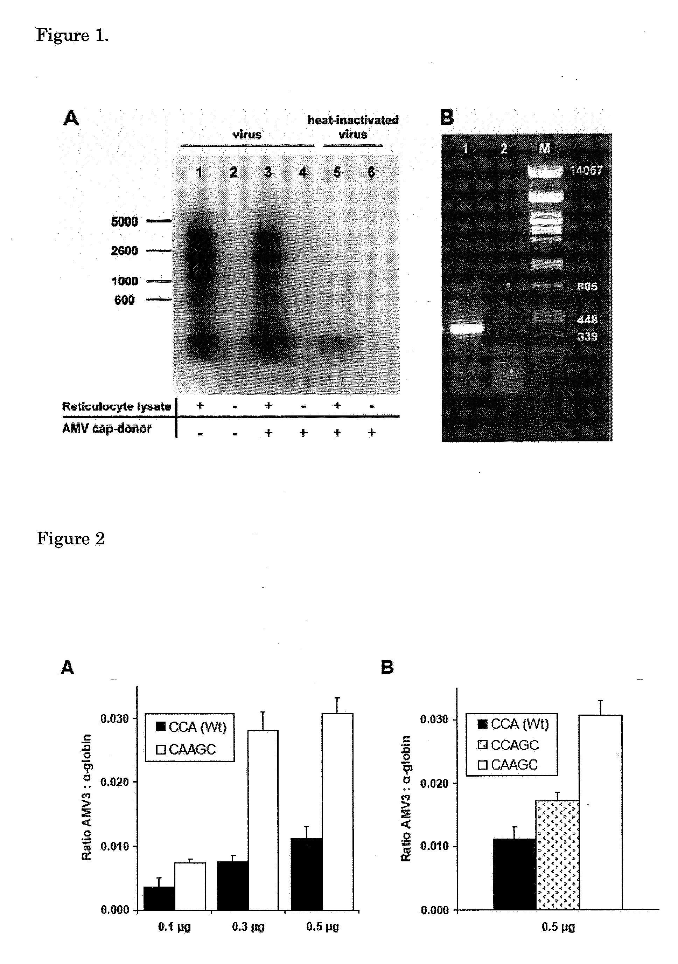

### Figure 1. Analysis of in vitro synthesized Influenza mRNA

(A) *In vitro* transcription reactions using radiolabelled nucleotides in the presence/absence of RRL. Lines 1-4: Active virus was used. Lines 5-6: heat-inactivated virus was used as control. In the absence of RRL, addition of AMV3 capped leader molecules did not recover transcription.
(B) RT-PCR analysis of *in vitro* synthesized *Influenza* mRNA in the presence of RRL. Active virus was used in lane 1 and heat-inactivated virus in lane 2. Reverse transcription was performed using internal primer specific for the Influenza NS1 gene, followed by PCR using internal primer for the same gene in combination with a primer specific for the 5' first 11 nucleotides of α-globin mRNA.

### Figure 2. Quantification of AMV/α₋globin leader initiated NS1 transcription by two step real-time PCR analysis

*In vitro* synthesized mRNA in the presence of RRL and addition of (mutant) AMV3 capped leader, was reverse transcribed using an internal primer for *Influenza* NS1 gene. Quantitative real-time PCR amplification (on a 500-fold diluted cDNA) was performed by using a nested primer for the NS1 gene and a primer for the AMV3 or α-globin mRNA. Standard errors were calculated from three independent measurements.
(A) Active virus and increasing concentrations of C₁₀C₁₁A₁₂ (Wt) or C₁₀A₁₁A₁₂G₁₃C₁₄ were used (concentrations are indicated in the X-axis).
(B) Active virus and 0.5 µg of C₁₀C₁₁A₁₂, C₁₀C₁₁A₁₂G₁₃C₁₄ or C₁₀A₁₁A₁₂G₁₃C₁₄ were used.

### Detailed description

"Cap(ped)-leader sequence(s)" or "cap(ped)-leader(s)" or "leader sequence(s)" or cap(ped) RNA primers are herein defined as m⁷ G-capped RNA sequences that can be used to prime transcription of the viral genome. The 5' cap is found on the 5' end of an mRNA molecule and consists of a 7-methylguanosine residue connected to the RNA via an unusual 5' to 5' triphosphate linkage (7MeGpppN). It is referred to as a 7-methylguanosine cap, abbreviated m⁷ G-cap. After completion of transcription the viral RNA is provided with a non-viral (not originating from the virus itself) 5' cap structure and non-viral leader sequences. Cap-leaders can be of any origin like viral, animal, human or plant, but are mostly of host origin. The process of initiation of viral transcription with a cap-leader sequence is also referred to as "cap-snatching".

This cap-snatching is one of the key events in viral genome transcription. Although several reports addressed cap-leader sequences, the specific requirements for cap-leader sequences for Influenza genome transcription have still not been elucidated. The current inventors have now found the requirements for cap-leader sequences necessary for preferential usage during priming of transcription. This cap-leader comprises at least a three base complementarity to the 3' ultimate residues of the viral RNA.

The term "base complementarity" refers to the complementarity between the 5'-cap-leader sequences and the 3' viral RNA. The viral RNA is herein also referred to as the viral template or virus template sequence. The complementary bases bind to the 3' ultimate end of the viral RNA wherein the A, G and C residues of the cap-leader subsequently bind to the 3' ultimate U, penultimate C and following G (also referred to as viral 3' terminal 1, 2, and 3 residues). "Ultimate" is herein defined as viral 3' terminal RNA base residue. "Penultimate" is herein defined as the RNA base subsequent to the ultimate base pair, the second most terminal viral RNA 3' base residue. Although single or double pairing of complementary bases between cap-leader and virus have been described the triple base pairing of the current invention surprisingly results in a very strong (preferentially used) cap-leader.

The current invention further identifies that residues in immediate proximity of the three complementary bases are important for efficient initiation and priming of transcription. The positions of these residues are referred to as -1 for the base immediately 5' upstream of AGC and -2 for the base at the second position 5' upstream of the AGC in the cap-leader sequence. The examples show that an A or T at position -1 and an A, T, or G at position - 2 in the cap-leader results in a very strong privilege for use in priming and elongation.

The cap leader preferably has the sequence ^{7m}G (N)₆₋₁₀-(A/U/G)-A/U-AGC which is herein referred to as the "consensus sequence". The final AGC in the consensus is predominant and is also referred to as three base complementarity or triple complementarity.

The consensus sequence also identifies an optimal leader length. The experiments performed in the current invention allow for the identification of an optimal length of the leader sequence between 10 and 20 nucleotides, wherein the predominant AGC is preferably at position 10-12 nucleotides or 11-13 nucleotides from the cap ^{7m}G (^{7m}G (N)₇₋₈-(A/U/G)-A/U-AGC). In another embodiment of the invention the leader can optionally be extended resulting in the following consensus sequence ^{7m}G (N)₆₋₁₀-(A/U/G)-A/U-AGC-(N)_{6-17..} After binding to the viral RNA the leader is cleaved (most likely after the three base complementary A, G, or C) whereafter elongation starts with the three base complementary ACG terminus. This elongation can be blocked by modification of the three base complementary AGC terminus. Said modification preferably is the addition of a 3'-phosphate group at the 3'-terminus of the cap leader. Another possible modification areis a replacement of one or more ribose ring(s) (or when a DNA consensus sequence is used of the dideoxyribose) within the cap-leader with one or more morpholino ring(s). Such a modification confers resistance to vial endonucleases.

In a further embodiment of the invention the base complementarity can be extended to 4 or 5 or more bases complementary to the virus 3' RNA. The corresponding complementary sequences after the three base AGC are different for respectively Influenza A, B and C. Thus, if specific inhibition of one of those three genera is intended, it is preferable to use an extended cap-leader with a sequence specific for said virus. These specific sequences are indicated in the table below and the cap-leader sequences will be extended to 4, 5 or more base complementary after the three bases AGC with these sequences respectively.

| | | |
|---|---|---|
| *Orthomyxoviridae* | Infl. A | ***UUCGCDUCUGCU***-3' |
| | Infl. B | ***CCUGCUUUGCU***-3' |
| | Infl. C | ***CGUGCUUUUGCU*-**3' |

The alignment of cap-leaders to the viral RNA template by virtue of base pairing is the initial step during initiation/priming of transcription. This initiates viral endonuclease cleavage of the capped-leader 10-20 nucleotides from the m⁷ G-cap 5' end. The resulting cap-leaders are used by the virus to prime transcription , i.e. elongation of the cap-leader. The chain elongation is herein also referred to as transcription, elongation or incorporation.

"Viral genome transcription" can thus be divided in three steps: initiation (alignment/binding of the cap-leader also referred to as priming of transcription), endonuclease cleavage and elongation. It is obvious for a person skilled in the art that when cap-leaders of shorter length are used, the endonuclease cleavage in the first step of initiation can become unnecessary and direct priming can take place by binding of the cap-leader to the viral RNA after which elongation can take place. On the other hand, cap-leaders shorter than 9 nt are too short to prime transcription.

In another aspect the invention relates to modified cap-leaders of the invention comprising a blocking group. This modification allows the cap-leader to bind to the viral RNA and prime transcription but from that moment is being blocked from becoming cleaved by the viral endonuclease or from being elongated. A blocking group is herein defined as any group that will allow binding of the cap-leader to the viral RNA but inhibits subsequent steps during the process of transcription initiation. Two blocking groups are preferred,to convert cap-leaders into dysfunctional ones, i.e. phosphorothioate bonds and a 3'-phosphate terminus. Modification of the phosphodiester bonds within the cap-leader into phosphorothioate bonds will introduce nuclease resistance and thereby block cleavage of the cap-leader sequence whereas the presence of a 3'-phosphate terminus instead of a 3'-OH terminus (RNA polymerases need a 3'-OH terminus for elongation) group will block elongation by the viral RNA polymerase. In this embodiment the 3'-phosphate blocking preferably takes place at the 3' terminal C. This will give the following consensus ^{7m}G (N)₆₋₁₀-(A/U/G)-A/U-AGCp wherein p is a 3'-phosphate group which can be interchanged for any other blocking group with similar functionality. A person skilled in the art will appreciate the use of other blocking groups (known in the art) that will result in the same function i.e. inhibition of subsequent steps during the process of transcription initiation.

Other chemical modifications that may be applied to enhance stability (by increasing nuclease resistance), affinity and bioavailability of dysfunctional cap-RNA leaders as inhibitor of Influenza transcription initiation are phosphorodiamidate linkages instead of phosphorothioates or phosphodiester bonds, and morpholino rings instead of ribose rings.

Replacement of phosphodiester by phosphorothioates will result in a replacement of the oxygen on each phosphate for a sulphur atom. This replacement inhibits degradation of the RNA or DNA with the phosphorothioate bond.

Phosphorodiamidate bounds also confer endonuclease resistance to DNA and RNA.

Morpholino or phospohorodiamidate morpholino refer to chemicals containing a six-membered morpholino ring. When bound to the viral RNA the cap-leader containing the morpholino ring(s) will be more resistant to viral endonuclease cleavage and thus elongation is inhibited.

In a further aspect the invention provides for a method to modify the cap-leaders or a method for preparing modified cap-leaders. This is preferably done by a chemical reaction wherein a blocking group is added to the cap-leader or wherein DNA or RNA with one or more phosphorothioate bond(s) instead of phosphodiester bond(s) is produced. This can be any known method in the art. The blocking group preferably is a 3'-phosphate groups or one or more phosphorothioate bonds. A person skilled in the art will be able to produce such compounds with blocking groups. For example the 3' phosphate can be added via esterification of the cap-leader with phosphoric acid. It will be known to a skilled person in the art how to make RNA with phosphorothioate bonds. In a further embodiment the invention relates to a method to modify the cap-leaders or a method for preparing modified cap-leaders, wherein the blocking group is a phosphorodiamidate bond instead of phosphorothioates or phosphodiester bonds, and/or morpholino rings instead of a ribose rings. It will be known to a skilled person in the art how to make RNA with phosphorodiamidate bonds and/or morpholino rings.

The chemical synthesis of RNA can be carried out by conventional phosphotriester, phosphite or phosphoramidite chemistry, using solid phase techniques such as those described in 'Chemical and Enzymatic Synthesis of Gene Fragments--A Laboratory Manual' (ed. H. G. Gassen and A. Lang), Verlag Chemie, Weinheim (1982), For example chemically synthesis can be performed by using an DNA/RNA synthesizer and b-cyanoethyl chemistry.

In another aspect the invention provides for a method to inhibit viral genome transcription of a (-)ssRNA virus in a cell by providing said cell with a modified cap RNA leader which has at least three bases complementary to the 3'ultimate residues of the viral RNA wherein the modification consist of a modification on either of the three residues. Said virus is preferably Influenza and the 3'-ultimate residues of the virus have the sequence UCG. A "cell" is herein defined as any host cell that is or can be infected with the virus. The inhibition of viral RNA transcription in the current invention will result in an inhibition of the viral infection (and a general reduction of virus titers), since the virus is unable to replicate. The terms "inhibition of viral replication", "treatment of viral infections" and "inhibition of viral replication" herein, all refer to the initial inhibition of viral transcription.

The cap-leader can be applied to the cell using any known method used in the state of the art. The delivery of modified (dysfunctional) cap-leaders preferably takes place via delivery vehicles such as liposomes, polymers, viral particles (VP), virosomes, viral vectors, lipid particles or (degradable) nanoparticles. Most preferably virosomes, viral particles or liposomes containing Influenza H and N proteins are used, as these will specifically target the host cells (a.o. respiratory tract) that naturally become infected by Influenza virus.

Liposomes are herein referred to as vesicles composed of a bilayer membrane, wherein said bilayer typically could be a phospholipid and cholesterol bilayer. Liposomes can be composed of several different kind of lipids for example naturally-derived phospholipids (phosphatidylethanolamine), or surfactant components like DOPE (dioleoylphosphatidylethanolamine). Properties of liposomes can vary depending on the lipid composition (cationic, anionic, neutral lipid species). Liposomes. have the ability to function as delivery vehicle for several different content like proteins, small molecules, drugs, compounds or nucleic acids like DNA or RNA. In the current invention said liposomes are used to deliver nucleic acids like DNA or RNA. Liposomal content can be delivered into the cell via fusion of the lipid bilayer with the cell membrane (but specific targeting or receptor mediated uptake can also be used). Liposomes can be produced via sonication of phospholipids in water. Liposomes of different sizes and different compositions can be made. Liposomes composed with opsonins and ligands to activate endocytosis in cell types, or liposomes with targeting ligands such as monoclonal antibodies (making an immunoliposome), vitamins, or specific antigens can be made. Targeted liposomes can target nearly any cell type in the body. In a preferred embodiment of the infection the liposomes comprise Influenza H and N proteins, as these will specifically target the host cells (a.o. cells in the respiratory tract) that naturally become infected by Influenza virus.
Virosomes are lipid bilayer vesicles containing viral glycoproteins derived from enveloped viruses. Virosomes are generally produced by extraction of membrane proteins and lipids from enveloped viruses with a detergent, followed by removal of this detergent from the extracted lipids and viral membrane proteins.Virosomes thus represent reconstituted empty virus envelopes, devoid of nucleocapsid and genetic material. In this way virosomes are unable to replicate. In contrast to liposomes, virosomes contain functional viral envelope glycoproteins in the phospholipid bilayer membrane. Virosomes can be used to deliver for example proteins, small molecules, drugs, compounds or nucleic acids like DNA or RNA. In the current invention said virosomes are used to deliver nucleic acids like DNA or RNA. In a preferred embodiment of the infection the virosomes comprise Influenza H and N proteins, as these will specifically target the host cells (a.o. cells in the respiratory tract) that naturally become infected by Influenza virus.

Virus like particles (VLPs) or Viral particles (VP) are composed of viral protein(s) of a virus. Preferably said proteins are embedded within a lipid bilayer. In this way the viral particles resemble the virus from which they were derived but lack all other viral components and are therefore not infectious. VPs can be used to deliver for example proteins, small molecules, drugs, compounds or nucleic acids like DNA or RNA. In the current invention said VPs are used to deliver nucleic acids like DNA or RNA. In a preferred embodiment of the infection the VPs comprise Influenza H and N proteins, as these will specifically target the host cells (a.o. cells in the respiratory tract) that naturally become infected by Influenza virus.

In a further aspect of the invention the modified cap-leader sequence of the invention is used to treat viral infections. The viral infection can be any (-)ssRNA virus, but preferably is a Influenza infection. This aspect of the invention comprises administration of a pharmaceutical vehicle or pharmaceutical composition comprising an active amount of the modified cap-leader. Optionally the pharmaceutical composition is comprised of the modified cap-leader in or on a delivery vehicle and a pharmaceutical excipient. Administration can be in any suitable form known in the state of the art and is not limited to pharmaceutical formulations like, pills, tablets, capsules, powders, lotions, ointments, soft-gels, gels, creams, transdermal patches, solutions, suspensions or inhalers (powders and nebulized solutions or suspensions). The above described pharmaceutical formulations can be applied orally (pills, tablets and capsules), nasal or pneumonial (via inhalation), topically, intramuscular or intravenous or subcutaneous (via injection). Preferred are injections and inhalations. Preparation and administration of the pharmaceutical compositions can be via any known method in the art (See also Martindale the complete drug reference, S Sweetman, 35the edition 2007).

The invention will be explained in more detail in the following, nonlimiting examples.

### Experimental part

### Materials and methods

### Virus

Influenza virus strain A/Puerto Rico/8/34 (H1/N1) was propagated and purified from embryonated chicken eggs. In brief, allantoic fluid was collected and cleared by low speed centrifugation for 10 min at 1000g and filtrated through a 0,45 µm-pore-size filter. The filtrate was layered onto 0,5 ml 60% (wt/vol) sucrose (in PBS) - 2,0 ml 20% (wt/vol) sucrose (in PBS) step gradients and centrifuged at 150,000g for 2 h at 4°C, using a SW41 rotor (Beckmann). Virus particles were recovered from the interface, adjusted to 20% (wt/vol) sucrose (in PBS), and loaded onto the top of a 12 ml 20 to 60% (wt/vol) continuous sucrose gradient (in PBS). After centrifugation at 150,000g for 16 h at 4°C in a SW41 rotor (Beckmann), the virus fraction was collected. Purified virus (10-15 µg/µl) was stocked in small aliquots at -80°C prior to use in transcription assays.

### Construction of plasmids

AMV RNA3 (AMV3) constructs and wild-type (wt-AMV3) were generated from plasmid pXO32NcoP3, which contains a full length cDNA clone of AMV3 (Neeleman et al., 1993). Wt-AMV3, Mut-2 and Mut-3 were PCR amplified by using primer AMV3-Rv (complementary to nt 339-313 of the AMV3 sequence) and primers wt-AMV3, mut2AMV3 or mut3-AMV3 respectively. Mut-N₁₄, an AMV3-Influenza NP mutant which could base pair over a stretch of 14 nt to the 3' end of the viral NS1 template, was amplified with primers MutN₁₄-AMV3 and NPUP2. AMV3 constructs that contained a point mutation at position 11 of the AMV3 sequence were made by first amplifying pXO32NcoP3 using primers AMV3-Rv and B₁₁A₁₂ (B = G, A or T). A second amplification was performed with primer T7prom1-AMV3 for extension of the AMV3 leader sequence with the T7 promoter sequence. Point mutants of AMV3 at nucleotide 11 are referred to as C₁₀G₁₁A₁₂, C₁₀A₁₁A₁₂ and C₁₀U₁₁A₁₂ (in which nt 11 was changed from the wild-type C residue into a G, A or T respectively). Similarly, AMV3 mutants that contained a mutation at either position 10 or 11 of the AMV3 sequence, and additionally the three (AGC) base pairing residues were made by first amplification of the same plasmid with primers AMV3-Rv and D₁₀C₁₁A₁₂GC (D = C, G, A or T) or E₁₁A₁₂GC (E = C, G, A or T) respectively. Second amplification was performed with primer T7prom2-AMV3 or T7prom1-AMV3 for AMV3 constructs with mutation at position 10 or 11 respectively. Point mutants of AMV3 at nt 10 or 11 with the additional three base pairing residues are referred to as G₁₀C₁₁A₁₂G₁₃C₁₄ (in which nt 10 was changed from the wild-type C residue into an G, and nts at positions 13 and 14 into a G and C respectively), A₁₀C₁₁A₁₂G₁₃C₁₄ and U₁₀C₁₁A₁₂G₁₃C₁₄ or C₁₀G₁₁A₁₂G₁₃C₁₄, C₁₀A₁₁A₁₂G₁₃C₁₄ and C₁₀U₁₁A₁₂G₁₃C₁₄ respectively. Mutant C₁₀C₁₁A₁₂G₁₃C₁₄ was made by changing only the nucleotides at positions 13 and 14 from the wild-type TT residues into a G and C respectively. Amplified PCR fragments were purified using the GFX PCR purification kit (Roche), restriction enzyme digested with *BcimHI*/*EcoRI* and ligated into pUC19 using T4 DNA ligase (Promega). Individual clones were verified by sequence analysis. Constructs to determine the leader length requirement were made by first amplifying pXO32NcoP3 with primer AMV3-Rv and primers C₇A₈, C₈A₉, C₉A₁₀, C₁₀A₁₁, C₁₁A₁₂, C₁₂A₁₃, C₁₃A₁₄, C₁₄A₁₅, C₁₅A₁₆, C₁₆A₁₇ or C₁₇A₁₈. Primer T7prom3-AMV3 was used in the second PCR amplification. For these constructs, capped transcripts were made directly from the purified PCR fragments without additional cloning.

Primer sequences are listed Table 6 (Supplemental Data).

### Synthesis of capped leaders

*In vitro* synthesis of capped RNA leaders was performed by using the Ambion T7 mMESSAGE mMACHINE kit according to the manufacturer's instruction. Transcription was performed in the presence of cap-analog m⁷G(5')ppp(5')G.

Prior to transcription each DNA template was linearized with *Eco*RI. T7 RNA polymerase promoter sequences were introduced upstream of the AMV3 leader sequence by PCR amplification as described above.

### In vitro Influenza viral transcription and AMV capped leader competition assays

*In vitro* Influenza transcription assays were performed using approximately 10 µg of purified Influenza in a final volume of 25 µl, in analogy as previously described for TSWV (van Knippenberg et al., 2002). These assays contained 4 mM Mg acetate, 1 mM of each NTP, 0.1% NP-40, 0.8 U/µl RNasin, 2.5 µl translation mix (amino acid mixture of 1:1:1-cys; -lys; -met), and were supplemented by the AP-Biotech Rabbit Reticulocyte Lysate system according to the manufacturer's procedures. The amount of AMV3 capped leaders added to the reactions is indicated in the Tables. After incubation for 2 hr at 37 °C, the reaction mixture was extracted with phenol-chloroform and the RNA ethanol precipitated.

### Analyses of AMV3-Influenza mRNA sequences

De novo synthesized Influenza NP or NS1 mRNAs containing capped 5' leader sequences derived from AMV3 were amplified by RT-PCR (Schmidt and Mueller, 1999; Schramm et al., 2000). First-strand cDNA was synthesized using an internal primer for the Influenza NP or NS1 gene (NPUP1 or NS1UP1 respectively) and in the presence of a template-switch oligonucleotide (TS-oligo). Under certain Mg²⁺ concentrations Superscript II did add a few C residues at the 3' end of the cDNA, which allowed the template-switch oligonucleotide, containing a few G-residues at its 3' end, to subsequently base pair to the first-strand cDNA. Superscript II recognized this oligonucleotide as a new template and switched from the viral RNA transcript to the oligonucleotide as template. As a result, the first-strand cDNA became extended with residues complementary to the template-switch oligonucleotide. Final PCR amplification of the first-strand cDNA, consisting of the complete 5' end of the NP or NS1 mRNA, extended at its 5' end with a few C residues and the sequence complementary to the template-switch oligonucleotide, then involved a nested NP or NS1 primer (primer NPUP2 or NS1UP2 respectively) and a primer matching the TS oligonucleotide and the first 11 nucleotides of the α-globin mRNA leader sequence (primer α-globin), or the AMV3 leader sequence (primer AMV3-leader1). In the case of the leader length requirement experiment, primer AMV3-leader2 was used instead of primer AMV3leader1 during the PCR amplification. PCR products of expected size were gel-purified using the GFX PCR purification kit (Roche), cloned into pGEM-T Easy (Promega) according to the manufacturer's procedures, and sequence analysed.

### Two step quantitative real-time PCR analysis

De novo synthesized NS1 transcripts were reverse transcribed into single-stranded (ss) cDNA as described earlier, using an internal primer for the NS1 gene (NS1UP1) and the TS-oligo primer. Afterwards, the first-strand cDNA was applied in a SYBR Green 1-based quantitative real-time PCR according to Zwart et al. (2007), with minor modifications: an annealing temperature of 50° C was used and template concentrations were determined using the standard curve method (RotorGene 6.0 software, Corbett Research). Template cDNA was diluted 500-fold. A PCR reaction was performed with the AMV3-leader1 and NS1UP2 primers to determine the AMV3-NS1 cDNA concentration in the sample. A separate PCR reaction was performed with the α-globin and NS1UP2 primers to determine the α-globin-NS1 cDNA concentration. The AMV3NS1 to α-globin-NS1 ratio and its standard error were then calculated as described by Zwart et al. (2007).

### Results

### Example 1

### Influenza A virus particles support genome transcription in the presence of RRL

Prior to the analysis of Influenza A virus cap donor requirements and in specific determine a base pairing requirement, an *in vitro* transcription assay was established (Materials and Methods), in analogy to TSWV (van Knippenberg et al., 2002), in which mature virus particles support transcription. *In vitro* reaction products from assays performed in the presence of radionucleotides were resolved by RNA electrophoresis and clearly showed that Influenza virus purified from embryonated eggs, supported the synthesis of RNA molecules in the presence of RRL, but hardly or not when absent from the reaction (Figure 1A), like what earlier was observed for TSWV. The addition of AMV RNA as primers for viral transcription *in vitro* (Plotch et al., 1979; Bouloy et al., 1980; Robertson et al., 1980; Plotch et al., 1981; Li et al., 1998 and 2001; van Knippenberg et al., 2002) did not recover transcriptional activity when RRL was absent from the reaction **(****Figure 1A****),** nor did higher rNTP concentrations (data not shown).

To verify that (part of) the *de novo* synthesized RNA molecules were products of transcriptional activity rather than replicational activity, NS1 mRNA molecules from the *in vitro* reaction were cloned by reverse transcription (RT)-PCR and sequence analyzed for the presence of 5' non-viral leader sequences, either corresponding to the leader sequence of α-globin mRNAs present from the RRL, or of those from exogenously added AMV3 cap donors. Since the 5' non-viral leader sequence of Influenza transcripts are only 9-13 nts, primers to these sequences would not be specific enough due to their short length. The selective RT-PCR strategy as earlier applied to amplify *de novo* synthesized TSWV transcripts could thus not be used (Duijsings et al, 1999 and 2001; van Knippenberg et al., 2002). Hence, an alternative strategy was applied to RT-PCR *de novo* synthesized Influenza transcripts in which use was made of a template-switch capacity from reverse transcriptase Superscript II (see Materials and Methods; Schmidt and Mueller, 1999; Schramm et al., 2000). Using this approach, products of expected size, that matched the NS1 transcript harboring a non-viral leader sequence, were observed **(****Figure 1B****, Lane 1**). Cloning and sequences analysis of these products confirmed the presence of α-globin RNA leader sequences at the 5' ends of *Influenza* NS1 transcripts (data not shown), demonstrating that the RNA molecules synthesized *in vitro* indeed were the result of transcription. In a similar way *de novo* synthesized NS1 transcripts harboring AMV3 leader sequences were RT-PCR amplified as confirmed by cloning and sequence analysis (**Table 2**). Control reactions using heat-inactivated virus always resulted in the absence of PCR products **(****Figure 1B****, Lane 2).**

As the AMV3 and α-globin RNA leader sequences at the 5' end of NS1 transcripts were similar in length to those earlier reported for Influenza transcripts, the *in vitro* transcription assay was further exploited to test exogenously added (mutant) AMV3 molecules as cap donors for Influenza A virus transcription.

### Example 2

### Influenza A virus prefers capped RNA leaders with more base complementarity to the viral RNA template

To analyze whether during transcription initiation, Influenza A prefers capped RNA leader molecules with more base complementarity to the viral RNA template, in analogy to what has earlier been demonstrated for TSWV (van Knippenberg et al., 2005), four AMV3 leader constructs **(Table 1)** were made that only differed in their base complementarity to the 3' end of the viral RNA template, and provided to the *in vitro* Influenza A virus transcription assay to be tested as cap donor in pair-wise competition. The first, single base pairing construct was represented by wild type (wt) AMV3, showing single base complementarity to the viral RNA template with the A residue at nucleotide position 12 downstream the 5' capped end. The second, double base pairing construct (denoted Mut-2) was derived from wt-AMV3 and harbored two base pairing (AG) residues at position 12 and 13 downstream the 5' capped end. A third, triple base pairing construct (denoted Mut-3) derived from wt-AMV3, harbored three base pairing (AGC) residues at position 12 to 14 downstream the 5' capped end. The fourth construct, denoted Mut-N₁₄, reflected a genuine Influenza A virus transcript, and consisted of the AMV3 leader sequence 5' of the Influenza A virus NP gene which harbored 14 nts complementary to the first 14 nts of the NS1 genomic RNA template (**Table 1**). To discriminate between wt-AMV3 and the three mutants, a marker nucleotide was additionally introduced in each of these constructs by changing the C₁₀ residue within the leader sequence into A, G or T respectively **(Table 1).**

The first *in vitro* competition was performed with wt-AMV3 and Mut-2. After *in vitro* Influenza transcription, RNA was extracted from the reaction and analyzed by RT-PCR using the template-switch approach. PCR fragments of expected sizes were cloned and sequenced to analyze *de novo* synthesized NS1 transcripts for the presence of capped RNA leader sequences from wt-AMV3 or Mut-2. The results showed that all clones contained marker nucleotide A in the leader sequence of the NS1 transcript (**Table 2**), indicative for the presence of the Mut-2 RNA leader sequence. Even when Mut-2 was offered in a ratio 10 times less than wt-AMV3 (**Table 2**), all NS1 transcript clones, except one of which the origin of the leader could not be identified, revealed the presence of the Mut-2 RNA leader sequence (**Table 2**), suggesting that Influenza A virus *in vitro* exhibits a preference for AMV3 Mut-2, i.e. for AMV3 leader sequences that show a higher base complementarity to the viral RNA template. In a second experiment, wt-AMV3 was tested as cap donor in pair-wise competition with Mut-14. When both RNA molecules were offered to the *in vitro* transcription assay in a 1:1 ratio (at 50 ng each and 500 ng each), a total of 35 out of 39 cloned NS1 sequences contained the marker nucleotide of Mut-N₁₄ and only 4 from wt-AMV3. In case wt-AMV3 and Mut-N₁₄ were offered in a 10:1 ratio, 21 out of 25 contained the leader sequence of Mut-N₁₄. The origin of the leader sequence from 4 additional clones could not be identified due to the absence of the marker nucleotide. Together, these results strongly suggested that Influenza A virus *in vitro* prefers capped RNA leader sequences with more base complementarity to the 3' end of the viral RNA template. To further substantiate these results, two additional *in vitro* transcription assays were performed in which Mut-2 was tested in pair-wise competition with Mut-3, and Mut3 with Mut-N₁₄, respectively. When Mut-2 and Mut-3 were provided in equimolar amounts, the RNA-leader sequence of Mut-3 was used two times more than that of Mut-2 (**Table 2**). Surprisingly, when Mut-3 and Mut-N₁₄ were offered to the *in vitro* transcription assay in a 1:1 competition, no clear preference for one of the leaders was observed (**Table 2**). In a 10:1 competition of Mut-3 and Mut-N14, the 5'-leader sequences of cloned NS1 transcripts also got close to an approximate 10:1 ratio and supporting the idea that no clear preference seemed to exist between Mut-3 and Mut-N14. The absence of a preference in this case may have been caused by a possible influence of 5' sequences upstream the base pairing residues of the capped RNA leader.

Further analysis of RNA leader sequence within all NS1 clones showed the presence of repetitive sequences in 9 clones, likely as a result of prime and re-alignment of capped leader sequences, and supporting the idea that base pairing is involved in the alignment of capped RNA leader sequences along the viral RNA template during priming of transcription.

Altogether the data strongly suggested that Influenza A virus *in vitro* exhibits a strong preference for capped RNA leaders of AMV3 with more base complementarity to the Influenza RNA template.

### Example 3

### Influence of nucleotides at the 3' terminus of a capped leader in cap-snatching

To analyze whether nucleotides within the capped RNA leader sequence, positioned just upstream of the 3' end residues involved in base pairing to the viral RNA template (A, AG, or AGC for wt-AMV3, Mut-2 and Mut-3 resp.), affect the efficiency of cap donor usage a set of (mutant) AMV3 constructs was made that harbored either a C (construct wt-AMV3, also referred to as C₁₀C₁₁A₁₂), A (C₁₀A₁₁A₁₂), G (C₁₀G₁₁A₁₂) or U (C₁₀U₁₁A₁₂) at the -1 position of the assumed (single) base pairing residue A12 (**Table 1**).

The nucleotide changes at position 11 were simultaneously used as a marker nucleotide to discriminate between leaders derived from wt-AMV3 and the 3 derived mutants. Synthetic capped transcripts of all four AMV3 constructs were offered in equimolar amounts to the *in vitro* assay and *de novo* synthesized NS1 gene transcripts were RT-PCR amplified, cloned and sequenced. Surprisingly, 18 out of the 32 clones analyzed contained the G nucleotide at position 11 (**Table 3**), indicating a preference for the C₁₀G₁₁A₁₂ sequence. A detailed look revealed that almost all (17) of these 18 clones, the first viral A residue was lacking, likely due an endonuclease cleavage immediately after the G residue and subsequent internal priming on the 3'-penultimate C residue of the viral template. Furthermore, 8 from these showed a repetitive sequence that may have resulted from a prime-and-realignment. From the remaining 14 (out of 32) clones, 8 clones contained the C₁₀A₁₁A₁₂ leader, 4 the C₁₀U₁₁A₁₂ leader and only 2 the C₁₀C₁₁A₁₂ (wt-AMV3) sequence. A closer look at the snatched leaders from mutant C₁₀A₁₁A₁₂ showed that 7 out of these 8 clones resulted from endonuclease cleavage downstream C₁₀An₁₁, and not after A₁₂, giving rise to a 3'CA-terminus. These clones thereby resembled two clones in which the leader sequence derived from mutant C₁₀C₁₁A₁₂ after cleavage downstream C₁₁A₁₂. Although all (mutant) single base pairing AMV3 molecules were used as cap donor, Influenza A virus *in vitro* clearly exhibited a preference for leader sequences harboring a 3' CGA, primarily leading to_ internal primingTo identify the nucleotide residues at position -1 and -2 just upstream the base pairing residues of the capped RNA leader sequence, that would promote its usage as capped leader without having internal priming, or a change in the position of endonuclease cleavage relative to the distance of the 5'cap-structure, a second set of AMV3 mutants was made (**Table 1**), all harboring three base pairing residues (AGC) at the 3' end of the capped RNA leader sequence of AMV3 and an A, G, C or U residue at position -1 or -2. The resulting constructs were referred to as C₁₀C₁₁A₁₂G₁₃C₁₄, C₁₀A₁₁A₁₂G₁₃C₁₄, C₁₀G₁₁A₁₂G₁₃C₁₄ and C₁₀U₁₁A₁₂G₁₃C₁₄, and C₁₀C₁₁A₁₂G₁₃C₁₄, A₁₀C₁₁A₁₂G₁₃C₁₄, G₁₀C₁₁A₁₂G₁₃C₁₄ and U₁₀C₁₁A₁₂G₁₃C₁₄ respectively. Again, the changed nucleotide at position 11 (-1 relative to the first base pairing residue) or 10 (-2 relative to the first base pairing residue) were simultaneously used as marker nucleotide.

When all cap donor molecules harboring a changed nucleotide at position -1 were offered in a multiple competition, 19 out of 40 NS1 mRNA clones contained a leader from mutant C₁₀A₁₁A₁₂G₁₃C₁₄ and 15 from C₁₀U₁₁A₁₂G₁₃C₁₄ (**Table 3**). Of the remaining six clones, 5 contained the leader from C₁₀G₁₁A₁₂G₁₃C₁₄ and only 1 from C₁₀C₁₁A₁₂G₁₃C₁₄. Furthermore, in case the leader sequence originated from the cap donor with a G at -1, internal priming was now only observed in 1 out of 5 clones. When C₁₀G₁₁A₁₂G₁₃C₁₄ was offered individually to an *in vitro* assay, 5 out of 22 retrieved NS1 clones showed the absence of the first viral A residue, and indicative for internal priming. The lower frequency of internal priming with C₁₀G₁₁A₁₂G₁₃C₁₄ in comparison to a very high frequency with C₁₀G₁₁A₁₂ was likely due to the extra base pairing residues present in C₁₀G₁₁A₁₂G₁₃C₁₄ that apparently forced proper alignment along the 3' first three residues of the viral RNA template. This observation again supports the idea that base complementarity of the capped leader to the viral template plays a major role during cap-snatching.

When all cap donor molecules harboring a altered nucleotide at position -2 (**Table 1**), i.e. two residues upstream the first base pairing A, were offered in a multiple competition, no clear preference between capped leaders with a G, A or U was found for this position, but a C residue appeared to be inefficient as this one only showed up in 1 out of 31 clones (**Table 3**).

### Example 4

### Pair-wise competition of a single and a triple base pairing capped RNA leader with reciprocal 5' upstream sequences

Based on the data described above, AMV3-derived single base pairing capped leader molecules harboring a 3' CAA were relatively well used in competitions involving other AMV3 single base pairing capped leader molecules but those harboring a 3' CCA not. To analyze whether this influence was also found in pair-wise competitions of these molecules with triple base pairing cap donor molecules, thereby underscore the importance of nucleotide sequences just upstream the putative base pairing residues, two additional pair-wise competition assays were performed. In the first one, two mutant AMV3 leader molecules referred to as C₁₀A₁₁A₁₂ and C₁₀C₁₁A₁₂G₁₃C₁₄ (**Table 1**), that differed in their base complementarity to the viral RNA template and one residue at position -1 just upstream the putative base pairing residues of the RNA leader sequence, were tested and in the second one their reciprocal leader variants, referred to as C₁₀Cₗ₁A₁₂ (identical to wt-AMV3) and C₁₀A₁₁A₁₂G₁₃C₁₄ **(Table 1).** In the first case, 14 out of 18 clones analyzed contained a leader from mutant C₁₀C₁₁A₁₂G₁₃C₁₄ and 4 from C₁₀A₁₁A₁₂. In the second case, almost all (18) of the 19 clones contained the leader sequence of C₁₀A₁₁A₁₂G₁₃C₁₄ and only one from C₁₀C₁₁A₁₂. These results strongly supported the idea that whereas nucleotide sequences within a capped leader are of importance for their usage as cap-donor, these are being overruled by the addition of sequences that extend the base complementary to the viral template.

### Example 5

### Leader length requirements for priming Influenza transcription

Several publications have described cleavage of capped eukaryotic RNAs by Influenza A virus transcriptase, generally at 9-15 nucleotides downstream the 5'-terminal cap structure (Bouloy et al., 1978; Plotch et al., 1979; Robertson et al., 1980). To more precisely define the optimal leader length, the *in vitro* transcription assay described here was exploited for this purpose. To this end, AMV3 constructs in which base pairing residues were located at various positions relative to the 5' capped end of the AMV3 molecule were made and offered simultaneously in a cap donor competition assay. All cap donor molecules offered contained a CAGC sequence, embedded within an oligo U stretch, to guarantee a proper alignment of the capped RNA leader along the viral RNA template by base pairing of the trinucleotide AGC. The shortest leader harbored the first base pairing A residue at position 8 from the 5' capped end whereas the longest leader harbored the first A base pairing at position 18 **(Table 1).** Transcripts of all leader mutants were simultaneously offered in equimolar amounts to an *in vitro* Influenza transcription assay. RT-PCR cloning and sequence analysis of *de novo* synthesized viral transcripts revealed that 9 out 19 transcripts contained capped leaders originating from AMV3 mutant C₉-A₁₀G₁₁C₁₂, and 5 out of 19 were originated from mutant C₁₀A₁₁G₁₂C₁₃ (**Table 4**), suggesting an optimal leader length of 10-11 nucleotides. Several clones revealed the presence of repetitive sequences, again pointing towards the occurrence of "prime-and-realign".

### Example 6

### Reduction of α-globin leader initiated Influenza A virus transcription by single and triple base pairing cap donor molecules

To analyze the competitor effect of single and triple base pairing capped leader molecules (by competition) on α-globin leader initiated Influenza A virus transcription, three selected capped leader constructs, C₁₀C₁₁A₁₂, C₁₀C₁₁A₁₂G₁₃C₁₄ and C₁₀A₁₁A₁₂G₁₃C₁₄ (**Table 1),** that differed in their base complementarity to the viral RNA template and/or one residue at position -1 just upstream the putative base pairing residue/-s of the RNA leader sequence, were individually offered in various concentrations to the *in vitro* Influenza transcription assay. The relative amount of α-globin leader initiated NS1 transcription was analyzed by RT-PCR and revealed that increasing amounts of C₁₀C₁₁A₁₂ reduced, by competition, globin leader initiated NS1 transcription to a lesser extent than C₁₀C₁₁A₁₂G₁₃C₁₄ and C₁₀A₁₁A₁₂G₁₃C₁₄ did (data not shown).

To quantify this effect, the ratio of AMV3-NS1 to α-globin-NS1 was determined by means of a two step quantitative real-time PCR. The *de novo* synthesized NS1 transcripts were first reverse transcribed into single-stranded cDNA and then PCR analyzed (see Materials and Methods). An almost three fold increase of the AMV3-NS1 to α-globin-NS1 ratio was observed when capped leader RNA molecules C₁₀A₁₁A₁₂G₁₃C₁₄ were used compared to C₁₀C₁₁A₁₂ (Wt-AMV3) (**Figure 2A**). When all three AMV3 capped leader mutants were compared, samples containing C₁₀A₁₁A₁₂G₁₃C₁₄ had the highest AMV3-NS1 to α-globin-NS1 ratio, followed by C₁₀C₁₁A₁₂G₁₃C₁₄ and then by C₁₀C₁₁A₁₂ **(****Figure 2B****).**

Altogether, this not only demonstrated the importance of base pairing as a selective criterion for cap donor molecules, but also supports the idea that such favorably used capped leader molecules might be attractive for further exploitation in antiviral drug design against Influenza A virus.

### Tables

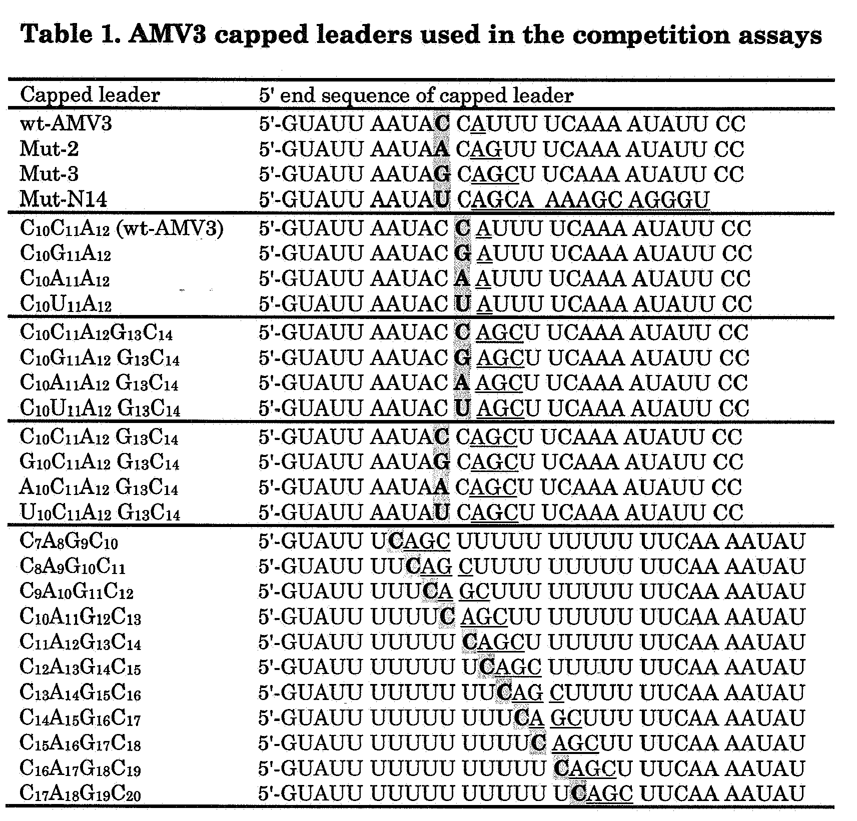

The nucleotide residues potentially base pairing to the viral template (3'-UCG...) are underlined; the marker nucleotide used to discriminate between the different AMV3 leaders is shaded.

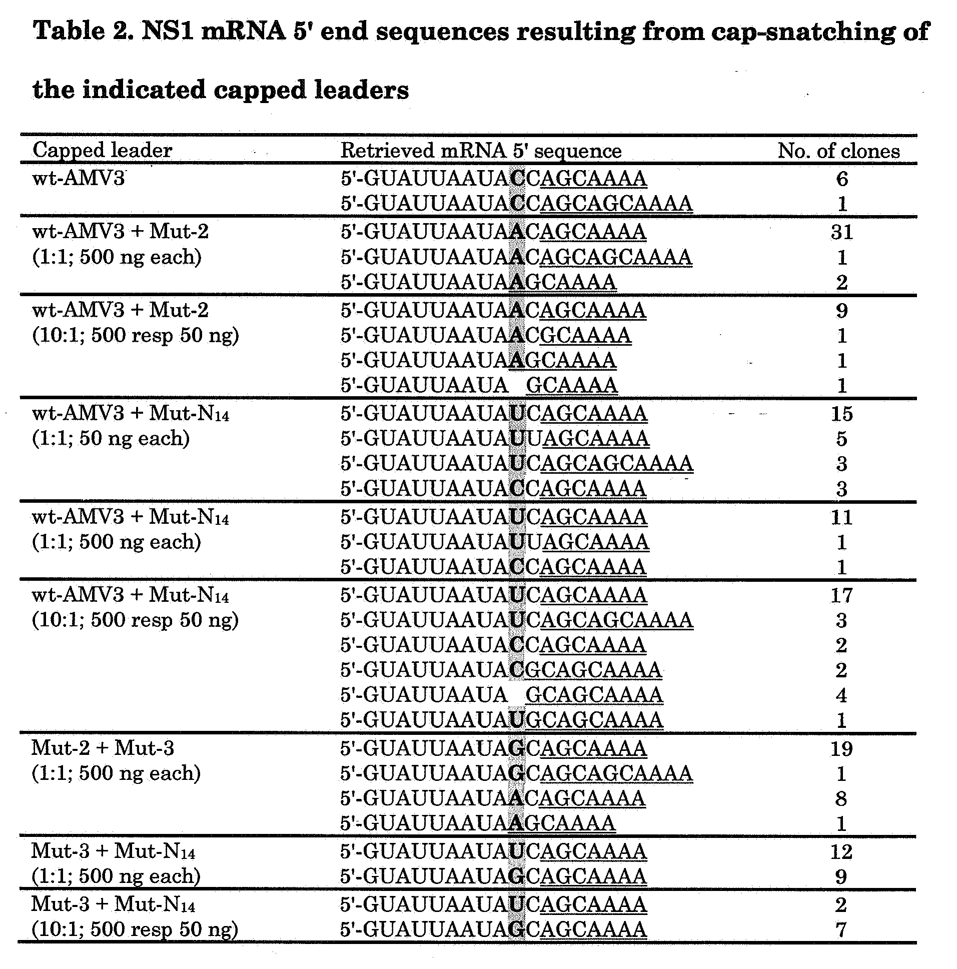

Influenza viral sequence is underlined; the marker nucleotide of each mutant is shaded.

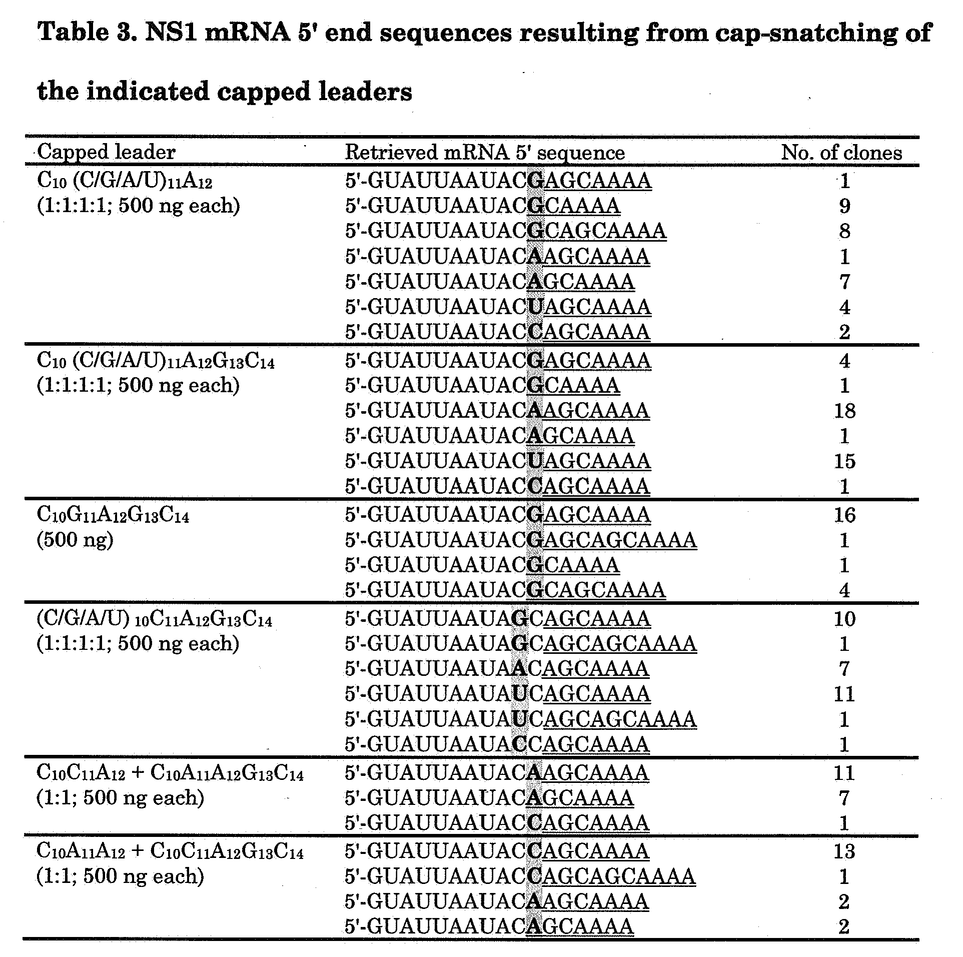

Influenza viral sequence is underlined; the marker nucleotide of each mutant is shaded.

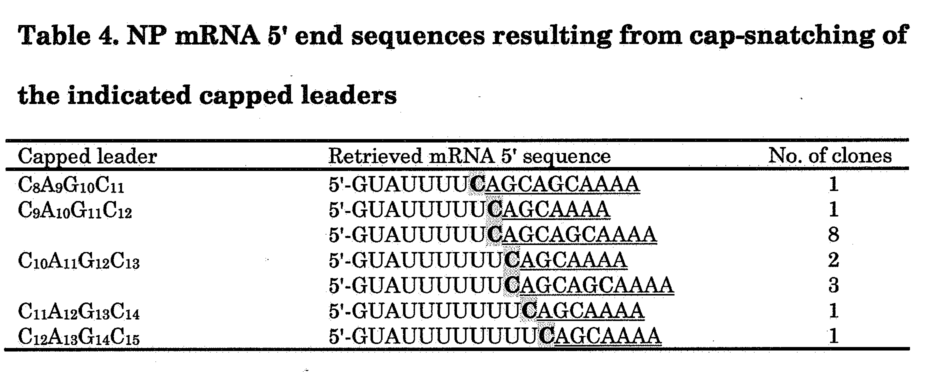

The nucleotide residues potentially base pairing to the viral template (3'-UCG...) are underlined; the marker nucleotide used to discriminate between the different AMV3 capped leaders is shaded.

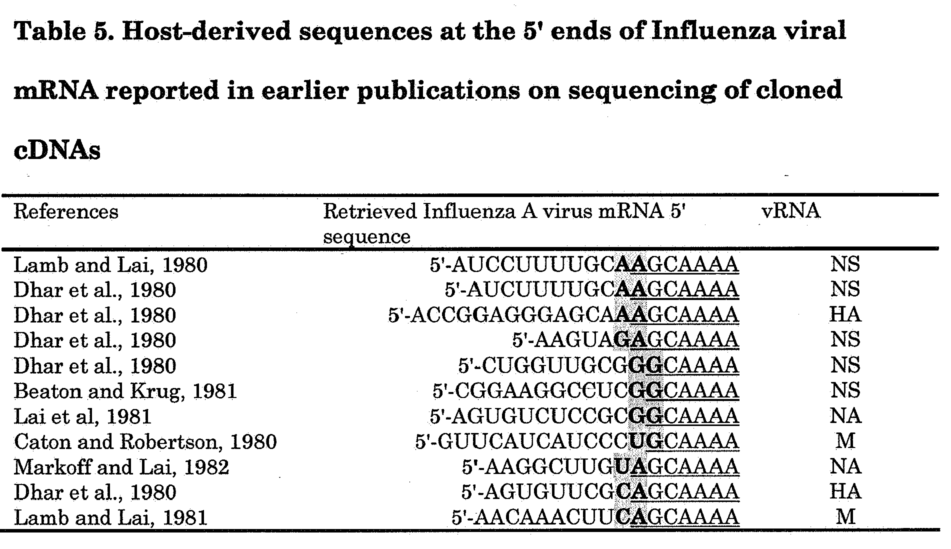

Influenza viral sequence is underlined; the 3'-terminal nucleotide of a capped leader which could potentially influence the use of the leader in the cap-snatching process is shaded. The proteins encoded by the cloned segments are indicated on the right.

### Supplemental Data

**Table 6. Primer sequences**

| Name | Primer sequence |
|---|---|
| TS-oligo | 5'-CCCGGATCCGGGGG |
| α-globin | 5'-CCCGGATCCGGGGGACACTTCTGGT |
| AMV3-leader1 | 5'-CCCGGATCCGGGGGG**TATTAATA** |
| AMV3-leader2 | 5'-CCCGGATCCGGGGGG**TATT** |
| NS1UP1 | 5'-CCCGAATTCGAGTCTCCAGCCGGTC |
| NS1UP2 | 5'-CCCGAATTCCGCCTGGTCCATTCTG |
| NPUP1 | 5'-CCCGAATTCGAGTCAGACCAGCCGTTG C |
| NPUP2 | 5'-CCCGAATTCGCTTGGCGCCAGATTCGC C |
| wt-AMV3 | 5'-CCCGGATCC**TAATACGACTCACTATA**GTATTAATACCATTTTCAAAATATTCC |
| Mut2-AM' | 5'-CCCGGATCC**TAATACGACTCACTATA**GTATTAATAACAGTTTCAAAATATTCC |
| Mut3-AM' | 5'-CCCGGATCC**TAATACGACTCACTATA**GTATTAATAGCAGCTTCAAAATATTCC |
| MutN₁₄-AMV3 | 5'-CCCGGATCC**TAATACGACTCACTATA**GTATTAATATCAGCAAAAGCAG |
| B₁₁A₁₂ | 5'-**CACTATA**GTATTAATACBATTTTCAAAATATTCC |
| D₁₀C₁₁A₁₂( | 5'-**CACTATA**GTATTAATADCAGCTTCAAAATATTCC |
| E₁₁A₁₂GC | 5'-**CACTATA**GTATTAATACEAGCTTCAAAATATTCC |
| C₇A₈ | 5'-**CGACTCACTATA**GTATTTCAGCTTTTTTTTTTTTTCAAAATATTCCAAT |
| C₈A₉ | 5'-**CGACTCACTATA**GTATTTTCAGCTTTTTTTTTTTTCAAAATATTCCAAT |
| C₉A₁₀ | 5'-**CGACTCACTATA**GTATTTTTCAGCTTTTTTTTTTCAAAATATTCCAAT |
| C₁₀A₁₁ | 5'-**CGACTCACTATA**GTATTTTTTCAGCTTTTTTTTTCAAAATATTCCAAT |
| C₁₁A₁₂ | 5'-**CGACTCACTATA**GTATTTTTTTCAGCTTTTTTTTCAAAATATTCCAAT |
| C₁₂A₁₃ | 5'-**CGACTCACTATA**GTATTTTTTTTCAGCTTTTTTTCAAAATATTCCAAT |
| C₁₃A₁₄ | 5'-**CGACTCACTATA**GTATTTTTTTTTCAGCTTTTTTCAAAATATTCCAAT |
| C₁₄A₁₅ | 5'-**CGACTCACTATA**GTATTTTTTTTTTCAGCTTTTTCAAAATATTCCAAT |
| C₁₅A₁₆ | 5'-**CGACTCACTATA**GTATTTTTTTTTTTTCAGCTTTTCAAAATATTCCAAT |
| C₁₆A₁₇ | 5'-**CGACTCACTATA**GTATTTTTTTTTTTTTCAGCTTTCAAAATATTCCAAT |
| C₁₇A₁₈ | 5'-**CGACTCACTATAGTA**GTATTTTTTTTTTTCAGCTTCAAAATATTCCAAT |
| T7prom1-AMV3 | 5'-CCCGGATCC**TAATACGACTCACTATA**GTATTAATAC |
| T7prom2-AMV3 | 5'-CCCGGATCC**TAATACGACTCACTATA**GTATT |
| T7prom3-AMV3 | 5'-CCCGGATCC**TAATACGACTCACTATA**GTATTT |
| AMV3-Rv | 5'-CCCGAATTCGAAGAGTACGAATTACGCG |

Residues which could potentially base pair to the viral template are underlined; bold residues are T7 promoter sequence.

### References

Beaton, A.R. and Krug, R.M., 1981, Nucl. Acids Res. 9:4423-4436.
Bishop, D.H.L. et al., 1983, Nucl. Acids Res. 11:6409-6418.
Bouloy, M. et al., 1990, Virology 175 :50-58.
Bouloy, M. et al., 1978, Proc. Natl. Acad. Sci. USA 75:4886-4890.
Bouloy, M. et al., 1980, Proc. Natl. Acad. Sci. USA 77:3952-3956.
Braam, J. et al., 1983, Cell 34:609-618.
Caton, A.J. and Robertson J.S., 1980, Nucl. Acids Res. 8:2591-2603.
Chan, A.Y. et al., 2006, Virology 351:210-217.
Chung, T.D. et al., 1994, Proc. Natl. Acad. Sci. USA 91:2372-2376.
Cianci, C. et al. 1995, J. Virol. 69:3995-3999.
Deng, T. et al., 2006, J. Virol. 80:2337-2348.
Dhar, R. et al. 1980, Cell 21:495-500.
Duijsings, D. et al., 1999, J. Virol. 73:5172-5175.
Engelhardt, O.G. et al., 2005, J. Virol. 79:5812-5818.
Fodor, E. et al., 1999, J. Viol. 73:9679-9682.
Fodor, E. et al., 2000, EMBO Rep. 1:513-518.
Garcin, D. Et al., 1995, J. Virol. 69:5754-5762.
Hagen, M. et al., 1994, J. Virol. 68:1509-1515.
Honda, A. et al., 1986, J. Biol. Chem. 261:5987-5991.
Huiet, L. et al., 1993, Virology 197:808-812.
Jin, H. and Elliot, R.M., 1993a, J. Virol. 67:1396-1404.
Jin, H. and Elliot, R.M., 1993b, J. Gen. Virol. 74:2293-2297.
Kormelink, R. et al., 1992, J. Gen. Virol. 73:2125-2128.
Krug, R.M. et al., 1979, Cell 18:329-334.
Krug, R.M. et al., 1980, Proc. Natl. Acad. Sci. USA 77:5874-5878.
Lai, C.J. et al., 1981, In: Genetic variation Among Influenza Viruses (D.P. Nayak, ed.), Academic Press, N.Y., p. 169-180.
Lamb, R.A. and Lai, C.J., 1980, Cell 21:475-485.
Lamb, R.A. and Lai, C.J., 1981, Virology 112:746-751.
Li, M.L. et al., 1998, EMBO J. 17:5844-5852.
Li, M.L. et al., 2001, EMBO J. 20:2078-2086.
Luo, G. Et al., 1997, J. Gen. Virol. 78:2329-2333.
Markoff, L. and Lai, C.J., 1982, Virology 119:288-297.
Neeleman, L. et al., 1993, Virology 196:883-887.
Neumann, G. et al., 1999, Proc. Natl. Acad. Sci. USA 96:9345-9350.
Peng, Q. et al., 1996, Virus Res. 42:149-158.
Pleschka, S., et al., 1996, J. Virol. 70:4188-4192.
Plotch, S.J. et al., 1979, Proc. Natl. Acad. Sci. USA 76:1618-1622.
Plotch, S.J. et al., 1981, Cell 23:847-858.
Rao, P. et al., 2003, EMBO J.22 :1188-1198.
Robertson, H.D. et al., 1980, Nucl. Acids Res. 8: 925-942.
Schmidt, W.M. and Müller, M.W., 1999, Nucl. Acids Res. 27:e31.
Schramm, G. et al., 2000, Nucl. Acids Res. 28:e96.
Shi, L. et al., 1995, Virology 208:38-47.
Shih, S.R. and Krug, R.M., 1996, Virology 226:430-435.
Simons, J.F. and Petterson, R.F., 1991, J. Virol. 65:4741-4748.
Van Knippenberg, I. Et al., 2002, Virology 303:278-286.
Van Knippenberg, I. et al., 2005, Virology 335:122-130.
Zwart, M.P. et al., 2007, J. Virol. Meth., in press.

## Claims

1. Cap-leader sequence which comprises at least three bases complementary to the 3'-ultimate residues of a (-)ssRNA virus template sequence.

2. A cap leader sequence according to claim 1 comprising the consensus sequence;
^{7m}G (N)₆₋₁₀-(A/U/G)-A/U-AGC, preferably ^{7m}G (N)₇₋₈-(A/U/G)-A/U-AGC

3. Modified cap-leader sequence according to claim 1 or 2 comprising a blocking group to avoid elongation by the viral polymerase.

4. Modified cap-leader according to claim 3 wherein said blocking group comprises a 3'-phosphate group

5. Modified cap-leader sequence according to claim 1 or 2 comprising a blocking group to avoid cleavage by the viral endonuclease.

6. Modified cap-leader according to claim 5 wherein said blocking group comprises a phosphorothioate bond, or a phosphorodiamidate bond.

7. Modified cap-leader according to claim 3 or 5 wherein said blocking group comprises one ore more morpholino rings.

8. Method for the modification of the leader sequence according to claim 1 or 2 comprising addition of the 3'-phosphate blocking group.

9. Method for preparing a modified cap-leader sequence according to claim 5 or 6 comprising replacement of at least one phosphodiester bond with at least one phosphorothioate bond, or phosphorodiamidate bond.

10. Method for preparing a modified cap-leader sequence according to claim 7 comprising replacement of at least one ribose ring with at least one morpholino ring.

11. Method to inhibit transcription of a (-)ssRNA virus in a cell by providing said cell with a modified cap-leader according to claims 3-7.

12. Method according to claim 11 wherein the virus is Influenza (A, B or C) and the 3'-ultimate residues have the sequence UCG.

13. Vehicle comprising a modified cap-leader sequence according to claims 3-7, wherein said vehicle is capable of introducing the cap-leader into the cell, preferably wherein said vehicle is a virosome, viral particle or liposome.

14. Pharmaceutical composition comprising an active amount of the modified cap-leader according to claims 3-7 or a vehicle according to claim 13, and a pharmaceutical excipient.

15. Modified cap-leader sequence according to claims 3-7 for use in the treatment of an (-)ssRNA virus infection

16. Use of the cap-leader sequence according to claims 3-7 to inhibit viral replication or to treat a viral infection.

17. Use of the method according to claims 11-12 to inhibit viral replication or to treat a viral infection.

18. Use of the vehicle or pharmaceutical composition according to claim 13 or 14 to inhibit viral replication or to treat a viral infection.

19. Use according to claims 16-18 wherein, the viral infection is an Influenza infection.
